# EUROPEAN PATENT APPLICATION

(11) **EP 4 663 195 A1**
(43) Date of publication of application: **17.12.2025**
(21) Application number: 24753412.6
(22) Date of filing: 07.02.2024
(51) Int. Cl.: A61K 35/17, A61P 17/00, A61P 37/06, A61P 43/00, C12N 5/0783

(54) **PHARMACEUTICAL COMPOSITION FOR TREATING OR PREVENTING PEMPHIGUS**

(30) Priority: 08.02.2023 JP 2023017733
(71) Applicant: Regcell Co., Ltd., Kyoto-shi, Kyoto 603-8436 (JP); Keio University, Tokyo, 108-8345 (JP); The University of Osaka, Osaka 565-0871 (JP); Kyoto University, Kyoto-shi, Kyoto 606-8501 (JP)
(72) Inventor: MIKAMI, Norihisa, Kyoto-shi, Kyoto 603-8436 (JP); SAKAGUCHI, Shimon, Suita-shi, Osaka 565-0871 (JP); AMAGAI, Masayuki, Tokyo 160-8582 (JP); TAKAHASHI, Hayato, Tokyo 160-8582 (JP)
(74) Representative: Vossius & Partner Patentanwälte Rechtsanwälte mbB
(86) International application number: PCT/JP2024/004214
(87) International publication number: WO 2024/166969

(57) **Abstract**

The present disclosure provides a medicament for treating or preventing pemphigus. More particularly, the present disclosure provides a pharmaceutical composition for treating or preventing pemphigus, the pharmaceutical composition containing, as an active ingredient, an induced regulatory T cell having at least one characteristic selected from the group consisting of CTLA4-positive, NT5E-positive, ITGAE (CD103)-positive, AREG-positive, CD172g-positive, and CD26-positive.

## Description

### Technical Field

The present disclosure relates to a pharmaceutical composition for treating or preventing pemphigus. More particularly, the present disclosure relates to a pharmaceutical composition containing regulatory T cells that specifically respond to desmoglein 3 (Dsg3).

### Background Art

As an important characteristic of CD25-positive CD4-positive regulatory T cells inherent in the immune system, the cells specifically express the transcription factor FoxP3, and a deficiency or mutation of FoxP3 may impair the development, differentiation, or suppressive function of the regulatory T cells. The regulatory T cells suppress the immune system by expression of various genes such as CTLA4 and IL-10 in addition to FoxP3. It is considered that epigenetic states such as DNA demethylation contribute to comprehensive gene expression regulation of regulatory T cells, including stable expression of FoxP3, and these states correlate with the functional phenotype of regulatory T cells.

### Summary of Invention

### Solution to Problem

The present inventors have found for the first time that when regulatory T cells are induced from human peripheral T cells, stable induced T cells are induced from the human peripheral T cells by stimulation using an anti-CD3 antibody, a resting culture is performed, a culture is performed again with anti-CD3 antibody stimulation, and then, a resting culture is performed again, such that regulatory T cells having high expression of suppressive molecules and a high suppressive function can be induced. In addition, the present inventors have also found that the inducible regulatory T cells thus obtained have sufficient immunosuppressive ability to function as a medicament for treating or preventing pemphigus.

Accordingly, the present disclosure provides the following.

### (Item 1)

A pharmaceutical composition for treating or preventing pemphigus, the pharmaceutical composition containing, as an active ingredient, an inducible regulatory T cell having at least one characteristic selected from the group consisting of CTLA4-positive, NT5E-positive, ITGAE (CD103)-positive, AREG-positive, CD172g-positive, and CD26-positive.

### (Item 2)

A pharmaceutical composition for treating or preventing pemphigus, the pharmaceutical composition containing, as an active ingredient, an inducible regulatory T cell that is NT5E-positive.

### (Item 3)

A pharmaceutical composition for treating or preventing pemphigus, the pharmaceutical composition containing, as an active ingredient, an inducible regulatory T cell that is ITGAE (CD103)-positive.

### (Item 4)

A pharmaceutical composition for treating or preventing pemphigus, the pharmaceutical composition containing, as an active ingredient, an inducible regulatory T cell that is AREG-positive.

### (Item 5)

A pharmaceutical composition for treating or preventing pemphigus, the pharmaceutical composition containing, as an active ingredient, an inducible regulatory T cell that is CD172g-positive.

### (Item 6)

A pharmaceutical composition for treating or preventing pemphigus, the pharmaceutical composition containing, as an active ingredient, an inducible regulatory T cell that is CD26-positive.

### (Item 7)

A pharmaceutical composition for treating or preventing pemphigus, the pharmaceutical composition containing, as an active ingredient, an inducible regulatory T cell that is CTLA4-positive.

### (Item 8)

The pharmaceutical composition according to any one of the preceding items, in which the inducible regulatory T cell is at least CTLA4-positive and FoxP3-positive.

### (Item 9)

The pharmaceutical composition according to any one of the preceding items, in which the inducible regulatory T cell is at least CD172g-positive and/or CD26-positive.

### (Item 10)

The pharmaceutical composition according to any one of the preceding items, in which the CNS2 region of the FOXP3 gene of the inducible regulatory T cell is demethylated.

### (Item 11)

The pharmaceutical composition according to any one of the preceding items, in which the inducible regulatory T cell is CD4-positive or CD8-positive.

### (Item 12)

The pharmaceutical composition according to any one of the preceding items, in which the inducible regulatory T cell is obtained from or derived from a human peripheral blood T cell or a human tissue-derived T cell.

### (Item 13)

The pharmaceutical composition according to any one of the preceding items, in which the inducible regulatory T cell is obtained by a method including:
(a) stimulating CD4-positive T cells or CD8-positive T cells in human peripheral blood with a first basal medium for about 1 to about 5 days;
(b) subjecting the cells obtained in the step (a) to a resting culture in a medium containing IL-2 for at least about 1 to about 3 days;
(c) stimulating the cells obtained in the step (b) with a second basal medium for about 1 to about 5 days; and
(d) subjecting the cells obtained in the step (c) to a resting culture in a medium containing IL-2 for at least about 1 to about 3 days.

### (Item 14)

The pharmaceutical composition according to any one of the preceding items, in which the pharmaceutical composition contains, as an active ingredient, a cell population containing the inducible regulatory T cells, and the cell population has a proportion of about 50% or more of cells each having at least one characteristic selected from the group consisting of FoxP3-positive, CTLA4-positive, NT5E-positive, ITGAE (CD103)-positive, AREG-positive, CD172g-positive, and CD26-positive.

### (Item 15)

A pharmaceutical composition for treating or preventing pemphigus, the pharmaceutical composition containing, as an active ingredient, a cell population containing inducible regulatory T cells, in which a proportion of NT5E-positive cells in the cell population is about 50% or more.

### (Item 16)

A pharmaceutical composition for treating or preventing pemphigus, the pharmaceutical composition containing, as an active ingredient, a cell population containing inducible regulatory T cells, in which a proportion of ITGAE (CD103)-positive cells in the cell population is about 50% or more.

### (Item 17)

A pharmaceutical composition for treating or preventing pemphigus, the pharmaceutical composition containing, as an active ingredient, a cell population containing inducible regulatory T cells, in which a proportion of AREG-positive cells in the cell population is about 50% or more.

### (Item 18)

A pharmaceutical composition for treating or preventing pemphigus, the pharmaceutical composition containing, as an active ingredient, a cell population containing inducible regulatory T cells, in which a proportion of CD172g-positive cells in the cell population is about 50% or more.

### (Item 19)

A pharmaceutical composition for treating or preventing pemphigus, the pharmaceutical composition containing, as an active ingredient, a cell population containing inducible regulatory T cells, in which a proportion of CD26-positive cells in the cell population is about 50% or more.

### (Item 20)

A pharmaceutical composition for treating or preventing pemphigus, the pharmaceutical composition containing, as an active ingredient, a cell population containing inducible regulatory T cells, in which a proportion of CTLA4-positive cells in the cell population is about 50% or more.

### (Item 21)

The pharmaceutical composition according to any one of the preceding items, in which proportions of at least the CTLA4-positive cells and FoxP3-positive cells in the cell population are each about 50% or more.

### (Item 22)

The pharmaceutical composition according to any one of the preceding items, in which proportions of at least the CD172g-positive cells and/or CD26-positive cells in the cell population are each about 50% or more.

### (Item 23)

The pharmaceutical composition according to any one of the preceding items, in which a proportion of cells having the at least one characteristic in the cell population is about 60% or more.

### (Item 24)

The pharmaceutical composition according to any one of the preceding items, in which a proportion of cells having the at least one characteristic in the cell population is about 80% or more.

### (Item 25)

The pharmaceutical composition according to any one of the preceding items, in which a proportion of FoxP3 strong-positive cells in the cell population is about 50% or more.

### (Item 26)

The pharmaceutical composition according to any one of the preceding items, in which the cell population contains about 90% or more T cells.

### (Item 27)

The pharmaceutical composition according to any one of the preceding items, in which the inducible regulatory T cells are contained in an amount of at least about 1 × 10³ cells per administration.

### (Item 28)

The pharmaceutical composition according to any one of the preceding items, in which the pharmaceutical composition is administered by injection.

### (Item 29)

The pharmaceutical composition according to any one of the preceding items, in which when the pharmaceutical composition is administered to a patient and is ineffective or insufficient, the pharmaceutical composition is additionally administered to the patient.

### (Item 30)

The pharmaceutical composition according to any one of the preceding items, in which the inducible regulatory T cell specifically responds to desmoglein 3 (Dsg3).

### (Item 31)

A method for treating or preventing pemphigus, the method including a step of administering, to a subject, the pharmaceutical composition according to any one of the preceding items.

### (Item 32)

A pharmaceutical composition containing, as an active ingredient, an inducible regulatory T cell that has at least one characteristic selected from the group consisting of CTLA4-positive, NT5E-positive, ITGAE (CD103)-positive, AREG-positive, CD172g-positive, and CD26-positive, and specifically responds to desmoglein 3 (Dsg3).

### (Item A1)

A method for treating or preventing pemphigus in a subject, the method including a step of administering, to the subject, an effective amount of inducible regulatory T cells having at least one characteristic selected from the group consisting of CTLA4-positive, NT5E-positive, ITGAE (CD103)-positive, AREG-positive, CD172g-positive, and CD26-positive.

### (Item A2)

A method for treating or preventing pemphigus in a subject, the method including a step of administering, to the subject, an effective amount of inducible regulatory T cells that are NT5E-positive.

### (Item A3)

A method for treating or preventing pemphigus in a subject, the method including a step of administering, to the subject, an effective amount of inducible regulatory T cells that are ITGAE (CD103)-positive.

### (Item A4)

A method for treating or preventing pemphigus in a subject, the method including a step of administering, to the subject, an effective amount of inducible regulatory T cells that are AREG-positive.

### (Item A5)

A method for treating or preventing pemphigus in a subject, the method including a step of administering, to the subject, an effective amount of inducible regulatory T cells that are CD172g-positive.

### (Item A6)

A method for treating or preventing pemphigus in a subject, the method including a step of administering, to the subject, an effective amount of inducible regulatory T cells that are CD26-positive.

### (Item A7)

A method for treating or preventing pemphigus in a subject, the method including a step of administering, to the subject, an effective amount of inducible regulatory T cells that are CTLA4-positive.

### (Item A8)

The method according to any one of the preceding items, in which the inducible regulatory T cells are at least CTLA4-positive and FoxP3-positive.

### (Item A9)

The method according to any one of the preceding items, in which the inducible regulatory T cells are at least CD172g-positive and/or CD26-positive.

### (Item A10)

The method according to any one of the preceding items, in which the CNS2 region of the FOXP3 gene of the inducible regulatory T cell is demethylated.

### (Item A11)

The method according to any one of the preceding items, in which the inducible regulatory T cells are CD4-positive or CD8-positive.

### (Item A12)

The method according to any one of the preceding items, in which the inducible regulatory T cells are obtained from or derived from human peripheral blood T cells or human tissue-derived T cells.

### (Item A13)

The method according to any one of the preceding items, in which the inducible regulatory T cells are obtained by a method including:
(a) stimulating CD4-positive T cells or CD8-positive T cells in human peripheral blood with a first basal medium for about 1 to about 5 days;
(b) subjecting the cells obtained in the step (a) to a resting culture in a medium containing IL-2 for at least about 1 to about 3 days;
(c) stimulating the cells obtained in the step (b) with a second basal medium for about 1 to about 5 days; and
(d) subjecting the cells obtained in the step (c) to a resting culture in a medium containing IL-2 for at least about 1 to about 3 days.

### (Item A14)

The method according to any one of the preceding items, in which a cell population containing the inducible regulatory T cells is contained as an active ingredient, and the cell population has a proportion of about 50% or more of cells each having at least one characteristic selected from the group consisting of FoxP3-positive, CTLA4-positive, NT5E-positive, ITGAE (CD103)-positive, AREG-positive, CD172g-positive, and CD26-positive.

### (Item A15)

A method for treating or preventing pemphigus in a subject, the method including a step of administering, to the subject, an effective amount of a cell population containing inducible regulatory T cells, in which a proportion of NT5E-positive cells in the cell population is about 50% or more.

### (Item A16)

A method for treating or preventing pemphigus in a subject, the method including a step of administering, to the subject, an effective amount of a cell population containing inducible regulatory T cells, in which a proportion of ITGAE (CD103)-positive cells in the cell population is about 50% or more.

### (Item A17)

A method for treating or preventing pemphigus in a subject, the method including a step of administering, to the subject, an effective amount of a cell population containing inducible regulatory T cells, in which a proportion of AREG-positive cells in the cell population is about 50% or more.

### (Item A18)

A method for treating or preventing pemphigus in a subject, the method including a step of administering, to the subject, an effective amount of a cell population containing inducible regulatory T cells, in which a proportion of CD172g-positive cells in the cell population is about 50% or more.

### (Item A19)

A method for treating or preventing pemphigus in a subject, the method including a step of administering, to the subject, an effective amount of a cell population containing inducible regulatory T cells, in which a proportion of CD26-positive cells in the cell population is about 50% or more.

### (Item A20)

A method for treating or preventing pemphigus in a subject, the method including a step of administering, to the subject, an effective amount of a cell population containing inducible regulatory T cells, in which a proportion of CTLA4-positive cells in the cell population is about 50% or more.

### (Item A21)

The method according to any one of the preceding items, in which proportions of at least the CTLA4-positive cells and FoxP3-positive cells in the cell population are each about 50% or more.

### (Item A22)

The method according to any one of the preceding items, in which proportions of at least the CD172g-positive cells and/or CD26-positive cells in the cell population are each about 50% or more.

### (Item A23)

The method according to any one of the preceding items, in which a proportion of cells having the at least one characteristic in the cell population is about 60% or more.

### (Item A24)

The method according to any one of the preceding items, in which a proportion of cells having the at least one characteristic in the cell population is about 80% or more.

### (Item A25)

The method according to any one of the preceding items, in which a proportion of FoxP3 strong-positive cells in the cell population is about 50% or more.

### (Item A26)

The method according to any one of the preceding items, in which the cell population contains about 90% or more T cells.

### (Item A27)

The method according to any one of the preceding items, in which the inducible regulatory T cells are contained in an amount of at least about 1 × 10³ cells per administration.

### (Item A28)

The method according to any one of the preceding items, in which the pharmaceutical composition is administered by injection.

### (Item A29)

The method according to any one of the preceding items, in which when the pharmaceutical composition is administered to a patient and is ineffective or insufficient, the pharmaceutical composition is additionally administered to the patient.

### (Item A30)

The method according to any one of the preceding items, in which the inducible regulatory T cell specifically responds to desmoglein 3 (Dsg3).

### (Item B1)

An inducible regulatory T cell for treating or preventing pemphigus, the inducible regulatory T cell having at least one characteristic selected from the group consisting of CTLA4-positive, NT5E-positive, ITGAE (CD103)-positive, AREG-positive, CD172g-positive, and CD26-positive.

### (Item B2)

An inducible regulatory T cell that is NT5E-positive for treating or preventing pemphigus.

### (Item B3)

An inducible regulatory T cell that is ITGAE (CD103)-positive for treating or preventing pemphigus.

### (Item B4)

An inducible regulatory T cell that is AREG-positive for treating or preventing pemphigus.

### (Item B5)

An inducible regulatory T cell that is CD172g-positive for treating or preventing pemphigus.

### (Item B6)

An inducible regulatory T cell that is CD26-positive for treating or preventing pemphigus.

### (Item B7)

An inducible regulatory T cell that is CTLA4-positive for treating or preventing pemphigus.

### (Item B8)

The inducible regulatory T cell according to any one of the preceding items, in which the inducible regulatory T cell is at least CTLA4-positive and FoxP3-positive.

### (Item B9)

The inducible regulatory T cell according to any one of the preceding items, in which the inducible regulatory T cell is at least CD172g-positive and/or CD26-positive.

### (Item B10)

The inducible regulatory T cell according to any one of the preceding items, in which the CNS2 region of the FOXP3 gene of the inducible regulatory T cell is demethylated.

### (Item B11)

The inducible regulatory T cell according to any one of the preceding items, in which the inducible regulatory T cell is CD4-positive or CD8-positive.

### (Item B12)

The inducible regulatory T cell according to any one of the preceding items, in which the inducible regulatory T cell is obtained from or derived from a human peripheral blood T cell or a human tissue-derived T cell.

### (Item B13)

The inducible regulatory T cell according to any one of the preceding items, in which the inducible regulatory T cell is obtained by a method including:
(a) stimulating CD4-positive T cells or CD8-positive T cells in human peripheral blood with a first basal medium for about 1 to about 5 days;
(b) subjecting the cells obtained in the step (a) to a resting culture in a medium containing IL-2 for at least about 1 to about 3 days;
(c) stimulating the cells obtained in the step (b) with a second basal medium for about 1 to about 5 days; and
(d) subjecting the cells obtained in the step (c) to a resting culture in a medium containing IL-2 for at least about 1 to about 3 days.

### (Item B14)

A cell population for treating or preventing pemphigus, the cell population containing inducible regulatory T cells, in which the cell population has a proportion of about 50% or more of cells each having at least one characteristic selected from the group consisting of FoxP3-positive, CTLA4-positive, NT5E-positive, ITGAE (CD103)-positive, AREG-positive, CD172g-positive, and CD26-positive.

### (Item B15)

A cell population for treating or preventing pemphigus, the cell population containing, as an active ingredient, a cell population containing inducible regulatory T cells, in which a proportion of NT5E-positive cells in the cell population is about 50% or more.

### (Item B16)

A cell population for treating or preventing pemphigus, the cell population containing inducible regulatory T cells, in which a proportion of ITGAE (CD103)-positive cells in the cell population is about 50% or more.

### (Item B17)

A cell population for treating or preventing pemphigus, the cell population containing inducible regulatory T cells, in which a proportion of AREG-positive cells in the cell population is about 50% or more.

### (Item B18)

A cell population for treating or preventing pemphigus, the cell population containing inducible regulatory T cells, in which a proportion of CD172g-positive cells in the cell population is about 50% or more.

### (Item B19)

A cell population for treating or preventing pemphigus, the cell population containing inducible regulatory T cells, in which a proportion of CD26-positive cells in the cell population is about 50% or more.

### (Item B20)

A cell population for treating or preventing pemphigus, the cell population containing inducible regulatory T cells, in which a proportion of CTLA4-positive cells in the cell population is about 50% or more.

### (Item B21)

The cell population according to any one of the preceding items, in which proportions of at least the CTLA4-positive cells and FoxP3-positive cells in the cell population are each about 50% or more.

### (Item B22)

The cell population according to any one of the preceding items, in which proportions of at least the CD172g-positive cells and/or CD26-positive cells in the cell population are each about 50% or more.

### (Item B23)

The cell population according to any one of the preceding items, in which a proportion of cells having the at least one characteristic in the cell population is about 60% or more.

### (Item B24)

The cell population according to any one of the preceding items, in which a proportion of cells having the at least one characteristic in the cell population is about 80% or more.

### (Item B25)

The cell population according to any one of the preceding items, in which a proportion of FoxP3 strong-positive cells in the cell population is about 50% or more.

### (Item B26)

The cell population according to any one of the preceding items, in which the cell population contains about 90% or more T cells.

### (Item B27)

The cell or cell population according to any one of the preceding items, in which the inducible regulatory T cells are contained in an amount of at least about 1 × 10³ cells per administration.

### (Item B28)

The cell or cell population according to any one of the preceding items, in which the cell or cell population is administered by injection.

### (Item B29)

The cell or cell population according to any one of the preceding items, in which when the cell or cell population is administered to a patient and is ineffective or insufficient, the pharmaceutical composition is additionally administered to the patient.

### (Item B30)

The cell or cell population according to any one of the preceding items, in which the inducible regulatory T cell specifically responds to desmoglein 3 (Dsg3).

### (Item C1)

Use of an inducible regulatory T cell for the manufacture of a medicament for treating or preventing pemphigus, the inducible regulatory T cell having at least one characteristic selected from the group consisting of CTLA4-positive, NT5E-positive, ITGAE (CD103)-positive, AREG-positive, CD172g-positive, and CD26-positive.

### (Item C2)

An inducible regulatory T cell that is NT5E-positive for use in the manufacture of a medicament for treating or preventing pemphigus.

### (Item C3)

Use of an inducible regulatory T cell that is ITGAE (CD103)-positive for the manufacture of a medicament for treating or preventing pemphigus.

### (Item C4)

Use of an inducible regulatory T cell that is AREG-positive for the manufacture of a medicament for treating or preventing pemphigus.

### (Item C5)

Use of an inducible regulatory T cell that is CD172g-positive for the manufacture of a medicament for treating or preventing pemphigus.

### (Item C6)

Use of an inducible regulatory T cell that is CD26-positive for the manufacture of a medicament for treating or preventing pemphigus.

### (Item C7)

Use of an inducible regulatory T cell that is CTLA4-positive for the manufacture of a medicament for treating or preventing pemphigus.

### (Item C8)

The use of the inducible regulatory T cell according to any one of the preceding items, in which the inducible regulatory T cell is at least CTLA4-positive and FoxP3-positive.

### (Item C9)

The use of the inducible regulatory T cell according to any one of the preceding items, in which the inducible regulatory T cell is at least CD172g-positive and/or CD26-positive.

### (Item C10)

The use of the inducible regulatory T cell according to any one of the preceding items, in which the CNS2 region of the FOXP3 gene of the inducible regulatory T cell is demethylated.

### (Item C11)

The use of the inducible regulatory T cell according to any one of the preceding items, in which the inducible regulatory T cell is CD4-positive or CD8-positive.

### (Item C12)

The use of the inducible regulatory T cell according to any one of the preceding items, in which the inducible regulatory T cell is obtained from or derived from a human peripheral blood T cell or a human tissue-derived T cell.

### (Item C13)

The use of the inducible regulatory T cell according to any one of the preceding items, in which the inducible regulatory T cell is obtained by a method including:
(a) stimulating CD4-positive T cells or CD8-positive T cells in human peripheral blood with a first basal medium for about 1 to about 5 days;
(b) subjecting the cells obtained in the step (a) to a resting culture in a medium containing IL-2 for at least about 1 to about 3 days;
(c) stimulating the cells obtained in the step (b) with a second basal medium for about 1 to about 5 days; and
(d) subjecting the cells obtained in the step (c) to a resting culture in a medium containing IL-2 for at least about 1 to about 3 days.

### (Item C14)

Use of a cell population for the manufacture of a medicament for treating or preventing pemphigus, the cell population containing inducible regulatory T cells, in which the cell population has a proportion of about 50% or more of cells each having at least one characteristic selected from the group consisting of FoxP3-positive, CTLA4-positive, NT5E-positive, ITGAE (CD103)-positive, AREG-positive, CD172g-positive, and CD26-positive.

### (Item C15)

Use of a cell population for the manufacture of a medicament for treating or preventing pemphigus, the cell population containing, as an active ingredient, a cell population containing inducible regulatory T cells, in which a proportion of NT5E-positive cells in the cell population is about 50% or more.

### (Item C16)

Use of a cell population for the manufacture of a medicament for treating or preventing pemphigus, the cell population containing inducible regulatory T cells, in which a proportion of ITGAE (CD103)-positive cells in the cell population is about 50% or more.

### (Item C17)

Use of a cell population for the manufacture of a medicament for treating or preventing pemphigus, the cell population containing inducible regulatory T cells, in which a proportion of AREG-positive cells in the cell population is about 50% or more.

### (Item C18)

Use of a cell population for the manufacture of a medicament for treating or preventing pemphigus, the cell population containing inducible regulatory T cells, in which a proportion of CD172g-positive cells in the cell population is about 50% or more.

### (Item C19)

Use of a cell population for the manufacture of a medicament for treating or preventing pemphigus, the cell population containing inducible regulatory T cells, in which a proportion of CD26-positive cells in the cell population is about 50% or more.

### (Item C20)

Use of a cell population for the manufacture of a medicament for treating or preventing pemphigus, the cell population containing inducible regulatory T cells, in which a proportion of CTLA4-positive cells in the cell population is about 50% or more.

### (Item C21)

The use of the cell population according to any one of the preceding items, in which proportions of at least the CTLA4-positive cells and FoxP3-positive cells in the cell population are each about 50% or more.

### (Item C22)

The use of the cell population according to any one of the preceding items, in which proportions of at least the CD172g-positive cells and/or CD26-positive cells in the cell population are each about 50% or more.

### (Item C23)

The use of the cell population according to any one of the preceding items, in which a proportion of cells having the at least one characteristic in the cell population is about 60% or more.

### (Item C24)

The use of the cell population according to any one of the preceding items, in which a proportion of cells having the at least one characteristic in the cell population is about 80% or more.

### (Item C25)

The use of the cell population according to any one of the preceding items, in which a proportion of FoxP3 strong-positive cells in the cell population is about 50% or more.

### (Item C26)

The use of the cell population according to any one of the preceding items, in which the cell population contains about 90% or more T cells.

### (Item C27)

The use of the cell or cell population according to any one of the preceding items, in which the inducible regulatory T cells are contained in an amount of at least about 1 × 10³ cells per administration.

### (Item C28)

The use of the cell or cell population according to any one of the preceding items, in which the cell or cell population is administered by injection.

### (Item C29)

The use of the cell or cell population according to any one of the preceding items, in which when the cell or cell population is administered to a patient and is ineffective or insufficient, the pharmaceutical composition is additionally administered to the patient.

### (Item C30)

The use of the cell or cell population according to any one of the preceding items, in which the inducible regulatory T cell specifically responds to desmoglein 3 (Dsg3).

In the present disclosure, it is intended that one or a plurality of features can be provided in further combination in addition to the specified combination. Note that still further embodiments and advantages of the present disclosure will be appreciated by those skilled in the art upon reading and understanding the following detailed description, if necessary.

Note that features and remarkable operations and effects of the present disclosure other than those described above will be apparent to those skilled in the art with reference to the following embodiments and drawings of the invention.

### Advantageous Effects of Invention

The regulatory T cells of the present disclosure can specifically respond to desmoglein, which is an antigenic protein of pemphigus. In addition, the regulatory T cells of the present disclosure highly express genes associated with regulatory T cells and have a strong suppression ability. Therefore, the regulatory T cells can be used as regulatory T cells for treatment, prevention, or the like of pemphigus.

In addition, the regulatory T cells of the present disclosure can be obtained by inducing functional regulatory T cells from human peripheral T cells in vitro. The regulatory T cells of the present disclosure have a high suppressive function and are stable as regulatory T cells. That is, the cell or cell population of the present disclosure specifically responds to desmoglein, and can stably express FoxP3, which is a master gene of regulatory T cells, using human peripheral T cells as a material, and thus is useful for treating or preventing pemphigus.

### Brief Description of Drawings

[FIG. 1] FIG. 1 is a schematic diagram showing a production example of Dsg3-specific S/F iTregs according to an embodiment of the present disclosure.
[FIG. 2] FIG. 2 is a diagram showing the results of flow cytometry analysis and bisulfite analysis of Dsg3-specific S/F iTregs according to an embodiment of the present disclosure.
[FIG. 3] FIG. 3 is a diagram showing the immunosuppressive effect of Dsg3-specific S/F iTregs according to an embodiment of the present disclosure.
[FIG. 4] FIG. 4 is a diagram showing the dermatitis-suppressive effect of Dsg3-specific S/F iTregs according to an embodiment of the present disclosure.
[FIG. 5] FIG. 5 is a diagram showing the dose-dependent effect of Dsg3-specific S/F iTregs on dermatitis inflammation according to an embodiment of the present disclosure. Body weight refers to (a change in) body weight, and Clinical score refers to a clinical treatment score.
[FIG. 6] FIG. 6 is a diagram showing the effect of suppressing production of inflammatory cytokines by Dsg3-specific S/F iTregs according to an embodiment of the present disclosure.
[FIG. 7] FIG. 7 is a diagram showing the long-term survival of Dsg3-specific S/F iTregs in a dermatitis model mouse according to an embodiment of the present disclosure.
[FIG. 8] FIG. 8 is a diagram showing the suppressive effect of Dsg3-specific S/F iTregs on a pemphigus model according to an embodiment of the present disclosure.
[FIG. 9] FIG. 9 is a diagram showing the results of flow cytometry analysis and bisulfite analysis of Dsg3-specific human S/F iTregs according to an embodiment of the present disclosure.
[FIG. 10] FIG. 10 is a diagram showing the immunosuppressive effect of Dsg3-specific human S/F iTregs according to an embodiment of the present disclosure.
[FIG. 11] FIG. 11 is a schematic diagram of treatment using Dsg3-specific S/F iTregs in Example 12. The Dsg3-specific B cell count was measured in the spleen and lymph nodes by an ELISpot method.
[FIG. 12] FIG. 12 shows the results of Example 12. The total B cell count is shown on the left and the Dsg3-specific B cell count is shown on the right. sLN indicates the sentinel lymph node and Spleen indicates the spleen.
[FIG. 13] FIG. 13 shows the results of titers and clinical severity of anti-Dsg3 antibodies by Dsg3-specific S/F iTregs in a pemphigus model. The Dsg3-specific S/F iTregs suppressed B cells in a Dsg3-specific manner, further suppressed Dsg3 antibody production and clinical symptoms of pemphigus, and therefore, are considered to be useful as an antigen-specific therapy.

### Description of Embodiments

Hereinafter, the present disclosure will be described while showing the best mode. Throughout the present specification, the expression of singular forms is to be understood as including the concept of plural forms unless otherwise stated. Therefore, the singular article (for example, "a", "an", "the", or the like in English) should be understood to include the concept of plural forms unless otherwise stated. In addition, the terms used in the present specification are to be understood as being used in the sense commonly used in the art unless otherwise stated. Therefore, unless defined otherwise, all technical and scientific terms used in the present specification have the same meaning as commonly understood by those skilled in the art to which the present disclosure pertains. In the case of conflict, the present specification (including definitions) will control.

Hereinafter, the definitions of the terms and/or basic technical concepts that are particularly used in the present specification will be described as appropriate.

As used herein, the term "about" means ±10% of the subsequent numerical value. For example, "about 20" is intended to include "18 to 22". The range of numerical values includes all values between the two endpoints and the values of the endpoints. The "about" in relation to a range applies to both endpoints of the range. Accordingly, for example, "about 20 to 30" includes "18 to 33".

As used herein, when a gene name and a product thereof are indicated, they may refer to both a gene and a protein when expressed in all capital letters, unlike normal usage. For example, FOXP3 gene and FOXP3 protein may be referred to separately, and when expressed as FoxP3, it indicates both the concept and the actual entity (as a whole) of the gene or the protein.

As used herein, the term "pemphigus" refers to an autoimmune blistering disease in which lesions are observed on the skin or mucous membranes. Pathologically, it is characterized by the formation of intraepidermal blisters caused by acantholysis resulting from impaired adhesion between epidermal cells, and immunopathologically, by autoantibodies against epidermal cell membranes deposited in the skin tissue or observed in the circulating blood. The target antigen protein of pemphigus is known to be desmoglein, which is a cadherin-type intercellular adhesion factor that plays an important role in epidermal intercellular adhesion.

Pemphigus is broadly classified into three types: pemphigus vulgaris, pemphigus foliaceus, and other types, and the other types include paraneoplastic pemphigus, proliferative pemphigus, which is a subtype of pemphigus vulgaris, erythematous pemphigus, which is a subtype of pemphigus foliaceus, herpetiform pemphigus, and drug-induced pemphigus.

As used herein, the term "regulatory T cell" refers to a T cell that is positive for FoxP3 expression. In the present specification, it may also be referred to as "Treg". Tregs include endogenous regulatory T cells (naturally occurring regulatory T cells: nTregs), inducible regulatory T cells (iTregs), and the like. Regulatory T cells usually can have various functions (for example, immunosuppressive functions).

As used herein, the term "inducible regulatory T cell (iTreg)" refers to a regulatory T cell that is negative for IKZF2 (Helios) expression. iTregs are usually obtained by inducing differentiation from naive CD4-positive T cells or the like.

As used herein, the term "naturally occurring regulatory T cell (nTreg)" refers to a regulatory T cell that is positive for the expression of IKZF2 (Helios) and CTLA4. It is usually a cell present in vivo.

As used herein, the term "peripheral T cell" refers to a T cell present outside the thymus, which can be obtained from peripheral blood, lymph nodes, or other tissues. As used herein, the term "peripheral T cell" means that the cell population only needs to contain peripheral T cells, and it is not necessary for the T cells to be isolated. In addition to T cells such as peripheral blood mononuclear cells (PBMCs), a cell fraction containing various lymphocytes may be used.

As used herein, the term "flow cytometry" refers to a technique for measuring the number of particles and the individual physical, chemical, and biological properties of cells, organisms, and other biological particles suspended in a liquid. An apparatus that uses this technique is referred to as a "flow cytometer". In the present disclosure, "positive" or "negative" of cell markers (for example, FoxP3, CTLA4, Helios, CD103, and the like) is determined by flow cytometry, as commonly used in the art. More specifically, in flow cytometry, cells are aligned in a single row and flowed, and the number of cells is counted using a spectroscopic method. For example, the number of target cells is counted by irradiating cells labeled with fluorescent or luminescent enzymes with laser light and detecting fluorescence or luminescent signals emitted from the cells by a detector such as a photodiode. In addition, the detection results from the detector can be input into a computer to generate and display a two-dimensional plot. Therefore, the presence or absence of target cells, the number thereof, and the like can be easily determined.

In the present specification, "demethylation" refers to removal of methyl modifications of adenine (for example, at the 6-position; m6A, or at the 1-position; m1A) or methylated cytosine (for example, at the 5-position; m5C, or at the 3-position; m3C). Demethylation can be specified using a technique known in the art, and can be measured using, for example, a bisulfite method.

In the present specification, the "cell population" is a group containing two or more cells, and for example, may be in a state where cells are gathered in a planar manner, or may be a cell mass formed by cells adhering to each other in a three-dimensional manner. In addition, the "cell population" may be formed of a single type of cell or may contain a plurality of types of cells.

As used herein, the term "stimulation" means stimulation via a TCR. For example, stimulation of T cells includes stimulation using an anti-CD3 antibody and a complex containing the same, stimulation using a peptide that binds to a TCR and a complex containing the peptide, stimulation using an antigen-presenting cell, stimulation using an anti-CD3 antibody and an antigen-presenting cell simultaneously, stimulation using a peptide or a protein and an antigen-presenting cell simultaneously, and the like.

As used herein, the term "resting culture" refers to culturing cells in a state without the stimulation described above.

### (Preferred embodiments)

Preferred embodiments of the present disclosure will be described below. The embodiments provided below are provided for a better understanding of the present disclosure, and the scope of the present disclosure should not be limited to the following description. Therefore, it is apparent that those skilled in the art can appropriately make modifications within the scope of the present disclosure in view of the description in the present specification. In addition, the following embodiments of the present disclosure can be used alone or in combination.

The present disclosure relates to a medicament application for treating or preventing pemphigus, containing highly functional and stable inducible regulatory T cells (also referred to as highly functional and stable iTregs or HSF iTregs).

In an aspect of the present disclosure, there is provided a pharmaceutical composition for treating or preventing pemphigus, the pharmaceutical composition containing, as an active ingredient, an inducible regulatory T cell having at least one characteristic selected from the group consisting of CTLA4-positive, NT5E-positive, ITGAE (CD103)-positive, AREG-positive, CD172g-positive, and CD26-positive. In another aspect of the present disclosure, there is provided a pharmaceutical composition containing, as an active ingredient, an inducible regulatory T cell that has at least one characteristic selected from the group consisting of CTLA4-positive, NT5E-positive, ITGAE (CD103)-positive, AREG-positive, CD172g-positive, and CD26-positive, and specifically responds to desmoglein 3 (Dsg3).

Pemphigus is an autoimmune blistering disease in which lesions are observed on the skin or mucous membranes. Pemphigus is characterized by the formation of intraepidermal blisters caused by acantholysis resulting from impaired adhesion between epidermal cells, and immunopathologically, by autoantibodies against epidermal cell membranes deposited in the skin tissue or observed in the circulating blood. The target antigen protein of pemphigus is known to be desmoglein, which is a cadherin-type intercellular adhesion factor that plays an important role in epidermal intercellular adhesion.

Pemphigus is broadly classified into three types: pemphigus vulgaris, pemphigus foliaceus, and other types, and the other types include paraneoplastic pemphigus, proliferative pemphigus, which is a subtype of pemphigus vulgaris, erythematous pemphigus, which is a subtype of pemphigus foliaceus, herpetiform pemphigus, and drug-induced pemphigus.

IgG autoantibodies observed in patients with pemphigus bind to desmoglein 1 (Dsg1) or desmoglein 3 (Dsg3). Desmoglein 1 or desmoglein 3 is a component of a calcium-binding transmembrane glycoprotein of a desmosome, which is an intercellular junction in a stratified squamous epithelium. Desmoglein plays an important role in the adhesion of epidermal keratinocytes to epidermal keratinocytes. Autoantibodies in pemphigus bind to desmogleins, thereby inhibiting the adhesion function of desmogleins and causing blisters in the epidermis as a result of the dissociation of epidermal cells.

Desmoglein 3 is a protein that is a member of the desmoglein family and the cadherin cell adhesion molecule superfamily, and preproprotein of desmoglein 3 undergoes proteolysis to be converted into a mature glycoprotein. The gene encoding desmoglein 3 is present as a gene cluster on chromosome 18 together with other members of the desmoglein gene family.

In an embodiment of the present disclosure, the inducible regulatory T cells of the present disclosure can specifically respond to desmoglein 3 (Dsg3) to suppress immunity and suppress skin inflammation.

In an embodiment of the present disclosure, the inducible regulatory T cells in the pharmaceutical composition of the present disclosure can have at least two characteristics selected from the group consisting of CTLA4-positive, NT5E-positive, ITGAE (CD103)-positive, and AREG-positive. In addition, in an embodiment of the present disclosure, the inducible regulatory T cells in the pharmaceutical composition of the present disclosure may be at least CTLA4-positive. Without intending to be limiting, the inducible regulatory T cells in the pharmaceutical composition of the present disclosure may be CD4-positive or CD8-positive.

In an embodiment of the present disclosure, the inducible regulatory T cells in the pharmaceutical composition of the present disclosure may be at least CTLA4-positive and FoxP3-positive. In an embodiment of the present disclosure, the inducible regulatory T cells in the pharmaceutical composition of the present disclosure may be at least CD172g-positive and/or CD26-positive.

The present disclosure provides a pharmaceutical composition for treating or preventing pemphigus, the pharmaceutical composition containing, as an active ingredient, an inducible regulatory T cell that is CD172g-positive. Since CD172g is a tyrosine kinase-associated protein involved in cell adhesion of neurons and the like (adhesion of cerebellar neurons, neurite outgrowth, and glial cell attachment), an inducible regulatory T cell that is CD172g-positive is expected to have activated functions related to adhesion and to exhibit high efficacy against pemphigus.

The present disclosure provides a pharmaceutical composition for treating or preventing pemphigus, the pharmaceutical composition containing, as an active ingredient, an inducible regulatory T cell that is CD26-positive. Since CD26 is a gene also known as DPP4, and is associated with glucose metabolism, insulin metabolism, and immune function (remarkable also in infectious diseases), an inducible regulatory T cell that is CD26-positive is expected to exhibit high efficacy against pemphigus.

The present disclosure provides a pharmaceutical composition for treating or preventing pemphigus, the pharmaceutical composition containing, as an active ingredient, an inducible regulatory T cell that is NT5E-positive. NT5E (CD73) is a cell membrane protein that has a catalytic activity to convert extracellular nucleotides into membrane-permeable nucleosides. The encoded protein is used as a determinant of lymphocyte differentiation. Defects in this gene may cause calcification of joints and arteries.

The present disclosure provides a pharmaceutical composition for treating or preventing pemphigus, the pharmaceutical composition containing, as an active ingredient, an inducible regulatory T cell that is ITGAE (CD103)-positive. ITGAE (CD103) encodes an α-integrin with an I domain and undergoes post-translational cleavage in the extracellular domain to produce a disulfide-linked heavy chain and light chain. This protein binds to β7 integrin to form an E-cadherin-binding integrin known as human mucosal lymphocyte-1 antigen. This protein is preferentially expressed on human intestinal intraepithelial lymphocytes (IELs), and may function as an accessory molecule for IEL activation, in addition to its role of adhesion. Therefore, an inducible regulatory T cell that is ITGAE (CD103)-positive is expected to have high efficacy against pemphigus.

The present disclosure provides a pharmaceutical composition for treating or preventing pemphigus, the pharmaceutical composition containing, as an active ingredient, an inducible regulatory T cell that is AREG-positive. AREG is a member of the epidermal growth factor family and is associated with epidermal growth factor (EGF) and transforming growth factor-a (TGF-α). This protein interacts with the EGF/TGF-α receptor to promote the growth of normal epithelial cells and inhibit the growth of certain aggressive cancer cell lines. In addition, it also functions in the development of mammary glands, ovum, and bone tissue. This gene is associated with a skin phenotype similar to psoriasis and is also associated with other pathological diseases including various types of cancers and inflammatory states.

The present disclosure provides a pharmaceutical composition for treating or preventing pemphigus, the pharmaceutical composition containing, as an active ingredient, an inducible regulatory T cell that is CTLA4-positive. CTLA4 is a protein that belongs to the immunoglobulin superfamily and transmits a suppressive signal to T cells. Membrane-bound CTLA4 functions as a homodimer linked by a disulfide bond, and soluble CTLA4 functions as a monomer. Mutations in this gene are said to be associated with insulin-dependent diabetes mellitus, Graves' disease, Hashimoto's thyroiditis, celiac disease, systemic lupus erythematosus, thyroid-associated orbitopathy, and other autoimmune diseases. Therefore, an inducible regulatory T cell that is CTLA4-positive is expected to have high efficacy against pemphigus.

The expression of FoxP3 in the regulatory T cells can be induced in a test tube by culturing CD4-positive T cells in a medium containing IL2 and TGFβ in the presence of an anti-CD3 antibody and an anti-CD28 antibody, and then culturing the cells in a medium containing IL2 and TGFβ. In addition, although several methods for inducing regulatory T cells from peripheral T cells are known, expression of FoxP3 in inducible regulatory T cells is unstable, and the expression of many functional molecules other than FoxP3 has not been confirmed.

In recent years, a culture method for inducing DNA demethylation by a method in which ascorbic acid is added to a medium in inducible regulatory T cells (Kasahara et al., Int. Immunol. (2017) 29(10):457-469) and a method in which CD28 antibody stimulation is not used (Mikami et al., Proc Natl Acad Sci U S A. (2020) 117(22):12258-12268) has been developed. However, even in the inducible regulatory T cells produced by these methods, the expression of functional molecules has not been confirmed, and sufficient immunosuppressive activity has not been obtained. Also in clinical trials, one clinical trial using inducible regulatory T cells has been conducted for GVHD, but the proportion of regulatory T cells in the cells used was low, and efficacy such as prevention of GVHD has not been recognized at present (MacMillan et al., Blood Adv. (2021) 5(5):1425-1436).

In the present disclosure, it is possible to provide a pharmaceutical composition containing, as an active ingredient, an inducible regulatory T cell in which the expression of FoxP3 is stable, and advantageously, an immunosuppressive action is stably maintained, and such inducible regulatory T cells can be produced, for example, by a method for producing inducible regulatory T cells as described elsewhere in the present specification. For example, the present disclosure can provide a pharmaceutical composition, in which inducible regulatory T cells are obtained by a method including: (a) stimulating CD4-positive T cells or CD8-positive T cells in human peripheral blood with a first basal medium for about 1 to about 5 days; (b) subjecting the cells obtained in the step (a) to a resting culture in a medium containing IL-2 for at least about 1 to about 3 days; (c) stimulating the cells obtained in the step (b) with a second basal medium for about 1 to about 5 days; and (d) subjecting the cells obtained in the step (c) to a resting culture in a medium containing IL-2 for at least about 1 to about 3 days.

The inducible regulatory T cells in the pharmaceutical composition of the present disclosure have an inducible regulatory T cell-specific demethylation state. The fact that the obtained regulatory T cells are in a regulatory T cell-specific demethylation state can be confirmed, for example, by the fact that the CNS2 region of the FoxP3 gene (FOXP3 (all in italics)) is demethylated. Since such a demethylation state can serve as an index of stability, the inducible regulatory T cells in the pharmaceutical composition of the present disclosure can be shown to be stable inducible regulatory T cells by confirming the demethylation state.

In the present description, the immunosuppressive activity or immunosuppressive action of the inducible regulatory T cells in the pharmaceutical composition of the present disclosure can be confirmed, for example, by measuring Cell Trace Violet intensity in responder T cells. The inducible regulatory T cells in the pharmaceutical composition of the present disclosure can stably provide an immunosuppressive activity or an immunosuppressive action, and, for example, the inducible regulatory T cells of the present disclosure can provide an immunosuppressive activity or an immunosuppressive action for at least about two weeks. The inducible regulatory T cells in the pharmaceutical composition of the present disclosure can have a higher immunosuppressive action than conventional regulatory T cells (including both induced and naturally occurring regulatory T cells). Therefore, the inducible regulatory T cells in the pharmaceutical composition of the present disclosure can also be referred to as functional or highly functional inducible regulatory T cells.

In an embodiment, the inducible regulatory T cells in the pharmaceutical composition of the present disclosure are capable of stably expressing FoxP3. Therefore, the inducible regulatory T cells in the pharmaceutical composition of the present disclosure can also be referred to as stable inducible regulatory T cells. The inducible regulatory T cells in the pharmaceutical composition of the present disclosure are, in an aspect, highly functional and stable, can be referred to as highly functional and stable inducible regulatory T cells (HSF iTregs).

In an embodiment of the present disclosure, whether or not markers in the inducible regulatory T cells of the present disclosure are positive can be determined by measuring a positivity rate using flow cytometry. For example, it is possible to determine whether or not the cells are positive based on a proportion of cells showing an antigen expression level equal to or higher than a reference standard by analyzing the cells with a flow cytometer. It can also be classified into negative, weakly positive, moderately positive, and strongly positive (weakly positive, moderately positive, and strongly positive collectively referred to as "positive") depending on the expression intensity of the cell surface markers. For example, depending on the setting of the instrument, a value of a median fluorescence intensity of each marker to a median fluorescence intensity of a negative control (stained with an isotype control antibody) can be classified as negative when less than 5, weakly positive when 5 or more and less than 10, moderately positive when 10 or more and less than 30, and strongly positive when 30 or more. Such determination can be made as exemplified in (Positivity Rate Measurement by Flow Cytometry), but the present disclosure is not limited thereto.

In an embodiment of the present disclosure, the expression intensity of cell surface markers in the inducible regulatory T cell of the present disclosure may be classified such that 10² or more is weakly positive, 10³ or more is moderately positive, and 10⁴ or more is strongly positive, 10³ or more is strongly positive and less than 10³ is weakly positive, or 10² or more is strongly positive and less than 10² is weakly positive, the expression intensity being determined, for example, based on analysis results obtained using a BD FACSLyric flow cytometer (BD Biosciences) using a sample prepared as described in Examples of the present application. Such determination can be appropriately set depending on experimental conditions, experimental purposes, the type of cells, instrument setting conditions, and the like, and the present disclosure is not limited thereto. In addition, even when analysis is performed using a flow cytometer other than the BD FACSLyric flow cytometer, it is possible to calculate the expression intensity on another instrument corresponding to the expression intensity measured by the BD FACSLyric flow cytometer, and the value indicating the expression intensity can be appropriately determined according to the instrument used.

In an embodiment of the present disclosure, the inducible regulatory T cells in the pharmaceutical composition of the present disclosure can be derived from any cells, and preferably can be derived from human peripheral blood T cells or human tissue-derived T cells.

In an embodiment of the present disclosure, the inducible regulatory T cells or the cell population thereof in the pharmaceutical composition of the present disclosure can target human cells, and can include inducible regulatory T cells or a cell population thereof induced by a predetermined technique using T cells obtained from human peripheral blood as a material. Therefore, in an embodiment of the present disclosure, the inducible regulatory T cells in the pharmaceutical composition of the present disclosure preferably include inducible regulatory human T cells. The properties of human cells and mouse cells are clearly different, and even when the same cell surface markers are present, the properties of the cells cannot be considered to be the same. For example, in the field of inducible regulatory T cells, the techniques that can be applied to mice and humans are different, and in the case of mice, most lymphocytes are antigen-unprimed, whereas in the case of humans, T cells in peripheral blood exhibit diverse degrees of antigen sensitization and activation. Therefore, in mice, it is possible to directly produce highly functional and stable inducible regulatory T cells from T cells collected from mouse lymphoid tissue; however, in humans, this is difficult, and cells cannot be regarded as equivalent based solely on the presence of cell surface markers.

In an embodiment of the present disclosure, the pharmaceutical composition of the present disclosure can contain a T cell population in which about 50% or more of the cells in the T cell population are inducible regulatory T cells as described elsewhere in the present specification. In an embodiment, the cell population of the present disclosure can contain inducible regulatory T cells as described elsewhere in the present specification in about 60% or more, about 65% or more, about 70% or more, about 75% or more, about 80% or more, about 85% or more, about 90% or more, about 95% or more, about 97% or more, or about 99% or more of the T cells in the cell population.

In an embodiment of the present disclosure, the T cells in the cell population of the present disclosure can be regulatory T cells. In this case, in the regulatory T cells of the cell population of the present disclosure, about 60% or more, about 65% or more, about 70% or more, about 75% or more, about 80% or more, about 85% or more, about 90% or more, about 95% or more, about 97% or more, or about 99% or more can be inducible regulatory T cells as described elsewhere in the present specification.

In an embodiment of the present disclosure, the cell population of the present disclosure may contain cells other than T cells, but preferably about 60% or more, about 65% or more, about 70% or more, about 75% or more, about 80% or more, about 85% or more, about 90% or more, about 95% or more, about 97% or more, or about 99% or more of the cell population are T cells, and more preferably about 90% or more are T cells.

In an aspect of the present disclosure, there is provided a pharmaceutical composition for treating or preventing pemphigus, the pharmaceutical composition containing, as an active ingredient, a cell population containing inducible regulatory T cells, in which proportions of CTLA4-positive cells and FoxP3-positive cells in the cell population are each about 50% or more. In an embodiment, the cell population in the pharmaceutical composition of the present disclosure can have proportions of the CTLA4-positive cells and FoxP3-positive cells of about 60% or more, about 65% or more, about 70% or more, about 75% or more, about 80% or more, about 85% or more, about 90% or more, about 95% or more, about 97% or more, or about 99% or more.

In an embodiment of the present disclosure, in the cell population in the pharmaceutical composition of the present disclosure, proportions of at least CD172g-positive cells and/or CD26-positive cells can each be about 50% or more. In an embodiment, the cell population in the pharmaceutical composition of the present disclosure can have proportions of the CD172g-positive cells and/or CD26-positive cells of about 60% or more, about 65% or more, about 70% or more, about 75% or more, about 80% or more, about 85% or more, about 90% or more, about 95% or more, about 97% or more, or about 99% or more.

In an embodiment of the present disclosure, in the cell population in the pharmaceutical composition of the present disclosure, a proportion of FoxP3 strong-positive cells can be about 50% or more. In an embodiment, the cell population in the pharmaceutical composition of the present disclosure can have a proportion of the FoxP3 strong-positive cells of about 60% or more, about 65% or more, about 70% or more, about 75% or more, about 80% or more, about 85% or more, about 90% or more, about 95% or more, about 97% or more, or about 99% or more. Although not intended to be limiting, in such a cell population in the pharmaceutical composition, measurement of the expression intensity of cell surface markers can be performed by measuring the positivity rate using flow cytometry as described above.

In another aspect of the present disclosure, there is provided a pharmaceutical composition for treating or preventing pemphigus, the pharmaceutical composition containing, as an active ingredient, a cell population containing inducible regulatory T cells, in which a proportion of FoxP3-positive cells in the cell population is about 50% or more. Although not intended to be limiting, in an embodiment, such a cell population in the pharmaceutical composition can contain FoxP3-positive inducible regulatory T cells in about 50% or more, about 60% or more, about 65% or more, about 70% or more, about 75% or more, about 80% or more, about 85% or more, about 90% or more, about 95% or more, about 97% or more, or about 99% or more on a cell count basis.

In another aspect of the present disclosure, there is provided a pharmaceutical composition for treating or preventing pemphigus, the pharmaceutical composition containing, as an active ingredient, a cell population containing inducible regulatory T cells, in which a proportion of CTLA4-positive cells in the cell population is about 50% or more. Although not intended to be limiting, in an embodiment, such a cell population in the pharmaceutical composition can contain CTLA4-positive inducible regulatory T cells in about 50% or more, about 60% or more, about 65% or more, about 70% or more, about 75% or more, about 80% or more, about 85% or more, about 90% or more, about 95% or more, about 97% or more, or about 99% or more on a cell count basis.

In another aspect of the present disclosure, there is provided a pharmaceutical composition for treating or preventing pemphigus, the pharmaceutical composition containing, as an active ingredient, a cell population containing inducible regulatory T cells, in which a proportion of NT5E-positive cells in the cell population is about 50% or more. Although not intended to be limiting, in an embodiment, such a cell population in the pharmaceutical composition can contain NT5E-positive inducible regulatory T cells in about 50% or more, about 60% or more, about 65% or more, about 70% or more, about 75% or more, about 80% or more, about 85% or more, about 90% or more, about 95% or more, about 97% or more, or about 99% or more on a cell count basis.

In another aspect of the present disclosure, there is provided a pharmaceutical composition for treating or preventing pemphigus, the pharmaceutical composition containing, as an active ingredient, a cell population containing inducible regulatory T cells, in which a proportion of ITGAE (CD103)-positive cells in the cell population is about 50% or more. Although not intended to be limiting, in an embodiment, such a cell population in the pharmaceutical composition can contain ITGAE (CD103)-positive inducible regulatory T cells in about 50% or more, about 60% or more, about 65% or more, about 70% or more, about 75% or more, about 80% or more, about 85% or more, about 90% or more, about 95% or more, about 97% or more, or about 99% or more on a cell count basis.

In another aspect of the present disclosure, there is provided a pharmaceutical composition for treating or preventing pemphigus, the pharmaceutical composition containing, as an active ingredient, a cell population containing inducible regulatory T cells, in which a proportion of AREG-positive cells in the cell population is about 50% or more. Although not intended to be limiting, in an embodiment, such a cell population in the pharmaceutical composition can contain AREG-positive inducible regulatory T cells in about 50% or more, about 60% or more, about 65% or more, about 70% or more, about 75% or more, about 80% or more, about 85% or more, about 90% or more, about 95% or more, about 97% or more, or about 99% or more on a cell count basis.

In another aspect of the present disclosure, there is provided a pharmaceutical composition for treating or preventing pemphigus, the pharmaceutical composition containing, as an active ingredient, a cell population containing inducible regulatory T cells, in which a proportion of CD172g-positive cells in the cell population is about 50% or more. Although not intended to be limiting, in an embodiment, such a cell population in the pharmaceutical composition can contain CD172g-positive inducible regulatory T cells in about 50% or more, about 60% or more, about 65% or more, about 70% or more, about 75% or more, about 80% or more, about 85% or more, about 90% or more, about 95% or more, about 97% or more, or about 99% or more on a cell count basis.

In another aspect of the present disclosure, there is provided a pharmaceutical composition for treating or preventing pemphigus, the pharmaceutical composition containing, as an active ingredient, a cell population containing inducible regulatory T cells, in which a proportion of CD26-positive cells in the cell population is about 50% or more. Although not intended to be limiting, in an embodiment, such a cell population in the pharmaceutical composition can contain CD26-positive inducible regulatory T cells in about 50% or more, about 60% or more, about 65% or more, about 70% or more, about 75% or more, about 80% or more, about 85% or more, about 90% or more, about 95% or more, about 97% or more, or about 99% or more on a cell count basis.

As described above, the properties of human cells and mouse cells are clearly different from each other, and therefore, even under the same induction conditions, the proportion of induction of specific cells differs between mouse cells and human cells. In the present disclosure, when induction is performed by a predetermined induction method, it is possible to obtain a cell population in which a proportion of positive cells for a predetermined cell surface marker is about 50% or more, and preferably to obtain a cell population in which "proportions of CTLA4-positive cells and FoxP3-positive cells are each about 50% or more".

CTLA4 is a molecule localized within a cell, and is not at a level that can be used, at least, as an index for enrichment or purification. In addition, since FoxP3 is a transcription factor and is entirely an intracellular molecule, FoxP3 cannot be detected on the surface at all and cannot be used for purification. That is, since CTLA4 and FoxP3 are markers expressed "within" T cells, it is not possible to obtain a cell population in which CTLA4-positive cells and/or FoxP3-positive cells account for about 50% or more by sorting and enriching using CTLA4 and/or FoxP3 as indices. Therefore, it is also not possible, according to conventional methods, to obtain a cell population in which proportions of CTLA4-positive cells and FoxP3-positive cells are each less than about 50%, and then, using such a cell population as starting material, to enrich by sorting with CTLA4 and/or FoxP3 as indices to obtain a cell population in which these cells each account for about 50% or more.

In an embodiment of the present disclosure, the inducible regulatory T cells in the pharmaceutical composition of the present invention mainly serve to exhibit the immunosuppressive function of Tregs, and when these cells account for more than half in a certain cell population, a medically effective immunosuppressive effect can be stably achieved, which is also important for technical and medical effects. That is, by containing 50% or more of T cells that exhibit a medically effective immunosuppressive effect, a stable cell preparation can be provided. Therefore, this effect is an extremely important point in medical practice, and is important not only as a difference in amount or numerical values but also as a quality issue regarding whether the preparation can be properly established.

In an embodiment of the present disclosure, in the cell population in the pharmaceutical composition of the present disclosure, the inducible regulatory T cells can further have characteristics of being ITGAE (CD103)-positive, NT5E-positive, and/or AREG-positive. These cell markers are genes that play an important role in immunosuppressive function, and since these markers are highly expressed, it is possible to maintain greater functional stability after administration of cells compared to conventional inducible regulatory T cells. For example, CD103 is important for migration of regulatory T cells to an inflammation site, NT5E (CD73) is important for production of adenosine having immunosuppressive ability, and AREG is important for tissue regeneration by regulatory T cells. The cell population of the present disclosure can achieve the effect of maintaining functional stability due to the high expression of these markers.

In an aspect of the present disclosure, a pharmaceutical composition containing the inducible regulatory T cells or the cell population of the present disclosure is provided. In addition, in another aspect, a regenerative medical material or product containing the inducible regulatory T cells or the cell population of the present disclosure is provided. These medicaments, regenerative medical materials, or products can be used together with media or any other additive used in the art. As such a medium, a medium obtained by adding necessary factors to a basal medium for animal cell culture used for culturing cells can be used. Examples of such media are described in detail elsewhere in the present specification. Examples of components added to the medium are also described in detail elsewhere in the present specification. When provided as such a product, DMSO or the like may be included.

In an embodiment, the pharmaceutical composition of the present disclosure can be administered by various routes of administration and dosages, but is preferably administered by injection. In an embodiment, the pharmaceutical composition of the present disclosure can contain inducible regulatory T cells as described elsewhere in the present specification, such that the inducible regulatory T cells are administered in an amount of at least about 1 x 10³ cells per administration.

In an embodiment, when the pharmaceutical composition of the present disclosure is administered to a patient and is ineffective or insufficient, the pharmaceutical composition is additionally administered to the patient. Whether the effect is absent or insufficient can be determined, for example, by using the degree of suppression of pemphigus symptoms as an index. In an embodiment, in the case of additional administration, it can be administered at least about one week, about two weeks, about three weeks, or about four weeks after the initial administration.

### (Production method)

The inducible regulatory T cells contained in the pharmaceutical composition of the present disclosure are highly functional and stable inducible regulatory T cells, and a method for producing such a pharmaceutical composition will be described in detail below in the present specification.

In an aspect of the present disclosure, there is provided a method for producing inducible regulatory T cells, the method including: (a) stimulating CD4-positive T cells or CD8-positive T cells in human peripheral blood with a first basal medium for about 1 to about 5 days; (b) subjecting the cells obtained in the step (a) to a resting culture in a medium containing IL-2 for at least about 1 to about 3 days; (c) stimulating the cells obtained in the step (b) with a second basal medium for about 1 to about 5 days; and (d) subjecting the cells obtained in the step (c) to a resting culture in a medium containing IL-2 for at least about 1 to about 3 days. In the present disclosure, the inducible regulatory T cells in the pharmaceutical composition of the present disclosure can be produced using any of CD4-positive T cells and CD8-positive T cells as starting material, and in an embodiment, the inducible regulatory T cells in the pharmaceutical composition of the present disclosure can also be produced using mixed cells of CD4-positive T cells and CD8-positive T cells as starting material.

In an aspect of the present disclosure, there is provided a method for producing a pharmaceutical composition for treating or preventing pemphigus, in which the pharmaceutical composition contains, as an active ingredient, an inducible regulatory T cell having at least one characteristic selected from the group consisting of FoxP3-positive, CTLA4-positive, NT5E-positive, ITGAE (CD103)-positive, AREG-positive, CD172g-positive, and CD26-positive, and the inducible regulatory T cell is obtained by a method including:
(a) stimulating CD4-positive T cells or CD8-positive T cells in human peripheral blood with a first basal medium for about 1 to about 5 days;
(b) subjecting the cells obtained in the step (a) to a resting culture in a medium containing IL-2 for at least about 1 to about 3 days;
(c) stimulating the cells obtained in the step (b) with a second basal medium for about 1 to about 5 days; and
(d) subjecting the cells obtained in the step (c) to a resting culture in a medium containing IL-2 for at least about 1 to about 3 days.

In an embodiment, the method of the present disclosure can also include: a step of culturing human peripheral T cells in a medium containing TGFβ and IL-2 in the presence of anti-CD3 antibody stimulation; a step of culturing the cells in a medium containing IL-2 in the absence of anti-CD3 antibody; and a step of culturing the cells again in a medium containing TGFβ and IL-2 in the presence of anti-CD3 antibody stimulation, such that regulatory T cells can be produced from human peripheral T cells (including CD4-positive T cells or CD8-positive T cells).

In an embodiment, the stimulation of T cells refers to the stimulation for obtaining Tregs, and is not particularly limited as long as it is stimulation via the TCR. For example, stimulation of T cells can include stimulation using an anti-CD3 antibody and a complex containing the same, stimulation using a peptide that binds to a TCR and a complex containing the peptide, stimulation using an antigen-presenting cell, stimulation using an anti-CD3 antibody and an antigen-presenting cell simultaneously, stimulation using a peptide or a protein and an antigen-presenting cell simultaneously, and the like, but the medium and conditions are not particularly limited as long as Tregs can be obtained.

In an embodiment, the medium and conditions for the resting culture are not particularly limited as long as the cells are cultured in a state without stimulation as described above.

As used herein, the term "anti-CD3 antibody stimulation" means that stimulation specific to a CD3 receptor on a cell is provided. Examples of the CD3 stimulation include an anti-CD3 agonist antibody. The anti-CD3 agonist antibody may be prepared by using a commercially available product as a research reagent or may be prepared by a conventional method. As the anti-CD3 antibody, for example, an antibody derived from an animal such as a mouse, a rabbit, a goat, or a cow, and an antibody derived from a human can be used.

In an embodiment, in the method of the present disclosure, once iTregs are induced (T cells are stimulated to induce demethylation) (step a), the cells are recovered by replacing the medium and performing a resting culture for 1 to 3 days (step b), and then, the T cells are stimulated again to induce demethylation (step c), thereby obtaining inducible regulatory T cells having functionality and stability. It is common technical knowledge that T cells exhibit apoptosis when T cells are generally stimulated twice; however, in the method of the present disclosure, by performing a "resting culture" and a "second stimulation", it has been found that inducible regulatory T cells having functionality and stability can be obtained without exhibiting apoptosis.

That is, in the method of the present disclosure, since the inducible regulatory T cells having functionality and stability can be obtained by performing T cell stimulation, a resting culture, and subsequent re-stimulation, the desired effect of the present disclosure can be achieved as long as the inducible regulatory T cells obtained in this manner or a cell population containing such inducible regulatory T cells is used. Therefore, in an embodiment of the present disclosure, the medium for culture and the type of stimulation are not particularly limited, and by using a medium having any composition and any type of stimulation, it is possible to obtain inducible regulatory T cells having functionality and stability.

In an embodiment, the medium used in each step may further contain retinoic acid and/or ascorbic acid. Preferably, the medium can contain ascorbic acid. In an embodiment, the medium may further contain a CDK8 inhibitor, a CDK19 inhibitor, and/or a CDK8/19 inhibitor. Preferably, the medium can contain a CDK8 inhibitor, a CDK19 inhibitor, and/or a CDK8/19 inhibitor.

In an embodiment of the present disclosure, the first basal medium and the second basal medium can each independently contain at least one, at least two, at least three, at least four, at least five or all of the factors selected from the group consisting of an anti-CD3 antibody, TGF-β1, IL-2, retinoic acid, a CDK8 inhibitor, a CDK19 inhibitor, a CDK8/19 inhibitor, and ascorbic acid. In addition, in another embodiment of the present disclosure, the first basal medium and the second basal medium can each independently contain an anti-CD3 antibody, TGF-β1, IL-2, retinoic acid, a CDK8 inhibitor, a CDK19 inhibitor, a CDK8/19 inhibitor, and ascorbic acid. The concentration of these respective components can be normal concentrations used in the art. In an embodiment, the concentrations of the CDK8 inhibitor, the CDK19 inhibitor, and/or the CDK8/19 inhibitor that may be used may be any suitable concentrations that may be used in the art, and for example, when SenexinA is used, the concentration may be about 0.1 µM or more, about 0.5 µM or more, about 1 µM or more, about 2 µM or more, about 3 µM or more, about 4 µM or more, about 5 µM or more, about 6 µM or more, about 7 µM or more, about 8 µM or more, about 9 µM or more, about 10 µM or more, about 12 µM or more, about 14 µM or more, about 16 µM or more, about 18 µM or more, about 20 µM or more, and the like, but is not limited to these concentrations, and can be appropriately changed by those skilled in the art depending on other medium compositions.

In an embodiment, the method of the present disclosure produces regulatory T cells from human peripheral T cells. The peripheral T cells include naive regulatory T cells, CD4-positive T cells, CD8-positive T cells, and the like. The regulatory T cells may be induced from a culture containing a plurality of types of T cells, or the regulatory T cells may be induced after specific cells such as CD4-positive T cells and CD8-positive T cells are isolated from these cells. In addition, regulatory T cells may be induced after T cells specific to a specific antigen are isolated. Thus, inducible regulatory T cells of the present disclosure include CD4-positive cells and CD8-positive cells. Note that, As used herein, the term "CD4-positive" or "CD4+" refers to a CD4-positive CD8-negative single-positive cell, unless otherwise specified. In addition, in the production method in the present disclosure, either CD4-positive or CD8-positive T cells can be used as starting material, and even after generation of inducible regulatory T cells, the CD4-positive or CD8-positive characteristics can be maintained unless special manipulation is performed.

In an embodiment, in the method of the present disclosure, the antibody may be added to the medium, or may be immobilized on an inner wall of a culture vessel or a surface of an insoluble carrier. The insoluble carrier can be a member capable of physically or chemically binding an anti-CD3 antibody, and can be insoluble in an aqueous solution. Examples of a material capable of physically adsorbing the anti-CD3 antibody include synthetic resins such as polystyrene, polyethylene terephthalate, polycarbonate, and polypropylene, and glass. The shape of the insoluble carrier is not particularly limited, and for example, a plate shape, a bead shape, a container shape, or the like can be adopted. The amount of the anti-CD3 antibody may vary depending on the titer or origin of the antibody used, but may be appropriately set so as to provide sufficient stimulation for induction of regulatory T cells.

In an embodiment, in the method of the present disclosure, a medium obtained by adding necessary factors to a basal medium for animal cell culture can be used for culturing the cells. Examples of the basal medium for animal cell culture that can be used in the method of the present disclosure include Iscove's modified Eagle's Medium, Ham's F12 medium, MEM Zinc Option medium, IMEM Zinc Option medium, IMDM medium, Medium 199, Eagle's Minimum Essential Medium (EMEM), αMEM medium, Dulbecco's modified Eagle's Medium (DMEM), RPMI 1640 medium, Fischer's medium, and a mixed medium or a medium partially modified in composition thereof.

The basal medium may contain serum (for example, fetal bovine serum (FBS)) or may be serum-free. The serum-free medium may optionally contain one or more serum substitutes, such as albumin, bovine serum albumin (BSA), transferrin, apotransferrin, KnockOut Serum Replacement (KSR) (a serum substitute for ES cell culture) (Thermo Fisher Scientific), N2 supplement (Thermo Fisher Scientific), B27 supplement (Thermo Fisher Scientific), fatty acids, insulin, collagen precursors, trace elements, 2-mercaptoethanol, 3'-thiolglycerol, and monothioglycerol. The basal medium may also contain one or more substances such as lipids (for example, chemically defined lipid concentrate), amino acids, L-glutamine, GlutaMAX (Thermo Fisher Scientific), non-essential amino acids (NEAA), vitamins (for example, nicotinamide, ascorbic acid), growth factors, antibiotics (for example, penicillin and streptomycin), antioxidants, pyruvate, buffers, inorganic salts, and equivalents thereof.

In one embodiment, as an example of the basal medium, RPMI 1640 medium containing serum and HEPES (4-(2-hydroxyethyl)-1-piperazineethanesulfonic acid) may be mentioned.

In the method of the present disclosure, the cells may be cultured under general animal cell culture conditions. A culture temperature is, but is not limited to, about 30 to 40°C, and is preferably about 37°C. The culture is preferably performed in an atmosphere of air containing CO₂, and the CO₂ concentration is preferably about 2 to 5%.

In the method of the present disclosure, the anti-CD3 antibody may be directly added to the medium, or an antibody immobilized on an inner wall of a culture vessel or on a surface of an insoluble carrier may be used. The amount of the anti-CD3 antibody may vary depending on the titer or origin of the antibody used, but may be appropriately set so as to provide sufficient stimulation for induction of regulatory T cells.

Examples of TGFβ used include TGFβ1, TGFβ2, and TGFβ3, and, for example, TGFβ1 may be used. The concentration of TGFβ may be appropriately determined by those skilled in the art, and is not particularly limited. When TGFβ1 or TGFβ3 is used as TGFβ, the concentration in the medium is not particularly limited, and may be 0.25 to 25 ng/mL, for example, about 10 ng/mL.

The concentration of IL-2 in the medium used is not limited, and can be about 5 U/mL to about 500 U/mL, for example, about 100 U/mL.

Retinoic acid and/or ascorbic acid may be further added to the medium used in the present disclosure. Preferably, the medium contains ascorbic acid. The concentration of ascorbic acid is not limited, and is about 1 to about 100 µg/mL, for example, about 10 µg/mL.

A CDK8 inhibitor, a CDK19 inhibitor, and/or a CDK8/19 inhibitor may be further added to the medium used in the present disclosure. Any CDK8 inhibitor, CDK19 inhibitor, and/or CDK8/19 inhibitor can be used in the present disclosure, and examples thereof include 4-[1-(2-methyl-1H-benzimidazol-5-yl)-1H-imidazo[4,5-c]pyridin-2-yl]-1,2,5-oxadiazol-3-amine, 3-{1-[1-(4-methoxyphenyl)piperidin-4-yl]-4-methyl-1H-imidazo[4,5-c]pyridin-2-yl}pyrazin-2-amine, or salts, hydrates, solvates thereof, and compounds described in US 8,598,344, WO 2013/001310 A, WO 2013/040153 A, WO 2013/116786 A, WO 2014/029726 A, WO 2014/063778 A, WO 2014/072435 A, WO 2014/090692 A, WO 2014/106606 A, WO 2014/123900 A, WO 2014/154723 A, WO 2014/194245 A, WO 2015/049325 A, WO 2015/100420 A, WO 2015/144290 A, WO 2015/159937 A, WO 2015/159938 A, WO 2016/009076 A, or WO 2018/139660 A. One example includes SenexinA or AS2863619, but the present disclosure is not limited thereto. The concentration of the CDK8 inhibitor, the CDK19 inhibitor, and/or the CDK8/19 inhibitor that may be used may be any suitable concentration that may be used in the art, and may be any suitable concentration described in the above literature, and can be appropriately changed by those skilled in the art according to other medium compositions.

In the method of the present disclosure, regulatory T cells can be induced by stimulating human peripheral T cells with an anti-CD3 antibody in a medium containing TGFβ and IL-2, and regulatory T cells having a high immunosuppressive function can be induced by stimulating the cells again with an anti-CD3 antibody after a resting culture in an IL-2-containing medium that does not contain an anti-CD3 antibody. It can be confirmed that the inducible regulatory T cells obtained have a high immunosuppressive function, for example, by comprehensive gene expression analysis using RNA sequencing, or by an in vitro cell proliferation inhibition assay.

In an embodiment of the present disclosure, the number of culture days in the step (a) of stimulating CD4-positive T cells or CD8-positive T cells in peripheral blood with a first basal medium for about 1 to about 5 days can be appropriately set by those skilled in the art, and is not particularly limited, but can be, for example, about 3 days.

In addition, in an embodiment, the number of culture days in the resting culture step in the step (b) can be appropriately set by those skilled in the art, and is not particularly limited, but can be, for example, about 2 days.

In addition, in an embodiment, the number of culture days in the culture step in the second basal medium in the step (c) can be appropriately set by those skilled in the art, and is not particularly limited, but can be, for example, about 3 days.

In addition, in an embodiment, the number of culture days in the resting culture step in step (d) can be appropriately set by those skilled in the art, and is not particularly limited, but can be, for example, about 2 days. In an embodiment, regulatory T cells having an inducible regulatory T cell specific demethylation state can be expanded by culturing in an unstimulated state in the presence of IL-2. The medium may further contain ascorbic acid, and by culturing in a medium further containing IL-2, a stable regulatory T cell culture of the inducible regulatory T cells can be obtained.

In order to isolate the regulatory T cells from the obtained cell culture containing the regulatory T cells, the cells may be isolated by a conventional method based on cell surface markers specific to the regulatory T cells, and for example, a FoxP3-positive fraction may be collected using a cell sorter. In addition, regulatory T cells having specific antigenic properties may also be isolated as desired.

The inducible regulatory T cells obtained by the method of the present invention are expected to be used for the treatment of human inflammatory diseases, for example, autoimmune diseases and allergies.

### (Measurement of positivity rate by flow cytometry)

In an embodiment, a positivity rate measurement by flow cytometry can be performed as follows.

### Preparation

· Fixation/Permeabilization Concentrate (hereinafter, Buffer) (eBio Science, 00-5123-43)
· Fixation/Permeabilization Diluent (hereinafter, Diluent) (eBio Science, 00-5223-56)
·Permeabilization Buffer (10×) (eBio Science, 00-833-56)
· FOXP3 Monoclonal Antibody (236 A/E7), PE (hereinafter, anti-FOXP3 antibody) (eBio Science, 12-4777-42)
· Mouse IgG1 kappa Isotype Control (P3.6.2.8.1), PE (PE control) (eBio Science)
· BV421, Mouse, Anti-Human, CD152 (hereinafter, anti-CTLA4 antibody) (BD)
· BV421 Mouse IgG2a, k Isotype Control (BV421 control) (BD)
· CD4 Monoclonal Antibody (RPA-T4), APC (hereinafter, anti-CD4 antibodies) (eBio Science)
· Mouse IgG1 kappa Isotype Control (P3.6.2.8.1), APC (hereinafter, APC control) (eBio Science)
· D-PBS (NACALAI TESQUE, INC., 14249-95)
· FBS (HyClone, SH30084.03)
·0.5 mol/l-EDTA solution (pH 8.0) (NACALAI TESQUE, INC., 06894-14)
· MilliQ water
· Centrifuge
· Safety cabinet
· Micropipette (P200, P1000)
·5 mL polystyrene round tube (hereinafter, dedicated tube) (Falcon, 352008)
· Nylon mesh (65 µm) (Kyoshin Riko, PP-65N)

### Reagent preparation

* Fixation Buffer (use 100 µL per sample)
   Buffer and Diluent are mixed at a ratio of 1 : 3.
* Perm Buffer
   Permeabilization Buffer (10×) is diluted 10-fold with MilliQ water.
* FACS Bufer (in the case of preparing 500 mL)
   D-PBS 489 mL
   FBS 10 mL (final concentration 2%)
   0.5 mol/l EDTA solution 1 mL (final concentration 1 mM)

The above reagents are stored at 4°C or on ice after preparation.

### Method

(1) Add 500 µL of FACS bufer to a 1.5 mL tube.
(2) Add 1 × 10⁶ cells of the final product to the tube of (1), and gently suspend using a micropipette.
(3) Centrifuge at 500 × g and 4°C for 5 minutes.
(4) After removing the supernatant with an aspirator, add 100 µL of fixation buffer and gently pipette. → Take care to avoid foaming.
(5) Fix by standing for 30 minutes or longer with light shielding on ice. → 45 minutes may also be acceptable.
(6) After fixation, add 1 mL of perm buffer to the tube and gently pipette.
(7) Prepare new 1.5 mL tubes according to the number of samples.
(8) Dispense 500 µL of each sample for control and for antibody staining.
(9) Centrifuge at 500 × g and 4°C for 5 minutes.
(10) During centrifugation, prepare an antibody preparation solution by diluting an anti-FOXP3 antibody (stock concentration = 0.05 mg/ml), an anti-CTLA4 antibody (Lot: 0030269, stock concentration = 0.2 mg/ml), and an anti-CD4 antibody (stock concentration = 0.1 mg/ml) 100-fold with perm buffer (hereinafter, referred to as the antibody preparation solution). For the control samples, prepare PE control (stock concentration = 0.1 mg/ml), BV421 control (stock concentration = 0.2 mg/ml), and APC control (stock concentration = 0.1 mg/ml) at the same concentration as the antibodies with the corresponding dyes (hereinafter, referred to as the control solution).
   * In the in-process control test, staining for CTLA4 is not performed.
(11) After removing the supernatant with an aspirator, add 100 µL of the antibody preparation solution to the antibody-staining sample, and 100 µL of the control solution to the control sample, and gently pipette. → Take care to avoid foaming.
(12) Fix by standing for 60 minutes with light shielding on ice.
(13) Start up the measurement equipment during staining.
(14) After staining, add 1 mL of FACS bufer and gently pipette.
(15) Centrifuge at 500 × g and 4°C for 5 minutes. → After removing the supernatant with an aspirator, the steps (14) and (15) are repeated once more for washing.
(16) After removing the supernatant with an aspirator, add 500 µL of FACS bufer and gently pipette.
(17) Place a nylon mesh on a dedicated tube using tweezers, and filter the suspension.
(18) Analyze using any flow cytometer. The number of cells collected for data acquisition should be 10,000 or more. In calculation of the positivity rate for the target antigen, the baseline is set so that the positivity rate of the control sample is 5% or less, and the proportion of cells showing an antigen expression level above the baseline is defined as the positivity rate.

### Remarks

· The reagents used for fixation and staining can also be purchased as a set of three under the name "Foxp3/Transcription Factor Staining Buffer Set" (Cat.: 00-5523).
· As long as the settings of the equipment and the like are not so deviated that it can be clearly determined from a general or scientific viewpoint that normal measurement cannot be performed, any settings may be used. A clear deviation is a case where various signals of the target cell population fall below the set fluorescence threshold value, a case where various signal values of the control sample or the measurement target sample fall below or exceed a limit value that can be normally measured by the equipment, or the like.

### (Measurement of immunosuppressive activity)

The cells of the present disclosure can have immunosuppressive activity. The immunosuppressive activity can be measured by various methods.

In an embodiment, as described in Examples, the suppression can be determined by measuring cell proliferation caused by the immune response of responder T cells. Preferably, in the present disclosure, it is desirable to demonstrate this using an in vivo model. For example, the activity can be measured by administering the inducible regulatory T cells of the present disclosure to a model mouse, collecting tissue after a certain period following administration, and determining whether immunosuppression is observed in the tissue. Confirmation of immunosuppression in the tissue can be achieved by various methods, and for example, the presence or absence of immunosuppression can be confirmed by histological staining analysis of the tissue.

### (General technique)

The molecular biological technique, the biochemical technique, and the microbiological technique used in the present specification are well known and commonly used in the art, and are described in, for example, Sambrook J. et al. (1989). Molecular Cloning: A Laboratory Manual, Cold Spring Harbor and its 3rd Ed. (2001); Ausubel, F.M. (1987). Current Protocols in Molecular Biology, Greene Pub. Associates and Wiley-Interscience; Ausubel, F.M. (1989). Short Protocols in Molecular Biology: A Compendium of Methods from Current Protocols in Molecular Biology, Greene Pub. Associates and Wiley-Interscience; Innis, M.A. (1990). PCR Protocols: A Guide to Methods and Applications, Academic Press; Ausubel, F.M. (1992). Short Protocols in Molecular Biology: A Compendium of Methods from Current Protocols in Molecular Biology, Greene Pub. Associates; Ausubel, F.M. (1995). Short Protocols in Molecular Biology: A Compendium of Methods from Current Protocols in Molecular Biology, Greene Pub. Associates; Innis, M.A. et al. (1995). PCR Strategies, Academic Press; Ausubel, F.M. (1999). Short Protocols in Molecular Biology: A Compendium of Methods from Current Protocols in Molecular Biology, Wiley, and annual updates; Sninsky, J.J. et al. (1999). PCR Applications: Protocols for Functional Genomics, Academic Press, the special volume of Experimental Medicine, "Experimental Methods for Gene Introduction & Expression Analysis", YODOSHA CO., LTD., 1997, and the like, and relevant parts (which may be all) of which are incorporated herein by reference.

For DNA synthesis techniques and nucleic acid chemistry for producing artificially synthesized genes, gene synthesis or fragment synthesis services such as GeneArt, GenScript, and Integrated DNA Technologies (IDT) can also be used, in addition, the DNA synthesis techniques and nucleic acid chemistry for producing artificially synthesized genes are described in, for example, Gait, M.J. (1985). Oligonucleotide Synthesis: A Practical Approach, IRL Press; Gait, M.J. (1990). Oligonucleotide Synthesis: A Practical Approach, IRL Press; Eckstein, F. (1991). Oligonucleotides and Analogues: A Practical Approach, IRL Press; Adams, R.L. et al. (1992). The Biochemistry of the Nucleic Acids, Chapman & Hall; Shabarova, Z. et al. (1994). Advanced Organic Chemistry of Nucleic Acids, Weinheim; Blackburn, G.M. et al. (1996). Nucleic Acids in Chemistry and Biology, Oxford University Press; Hermanson, G.T. (1996). Bioconjugate Techniques, Academic Press, and the like, and relevant parts (which may be all) of which are incorporated herein by reference.

In the present specification, "or" is used when "at least one or more" of the items listed in the text can be employed. The same applies to "or". When explicitly described in the present specification as "within the range" of "two values", the range also includes the two values themselves.

Reference literatures such as scientific literatures, patents, and patent applications cited in the present specification are incorporated herein by reference to the same extent that the entirety of each document is specifically described.

The present disclosure has been described above with reference to preferred embodiments for easy understanding. Hereinafter, the present disclosure will be described based on examples, but the above description and the following examples are provided for the purpose of illustration only and not for the purpose of limiting the present disclosure. Accordingly, the scope of the present disclosure is not limited to the embodiments or examples specifically described in the present specification, but is limited only by the claims.

### Examples

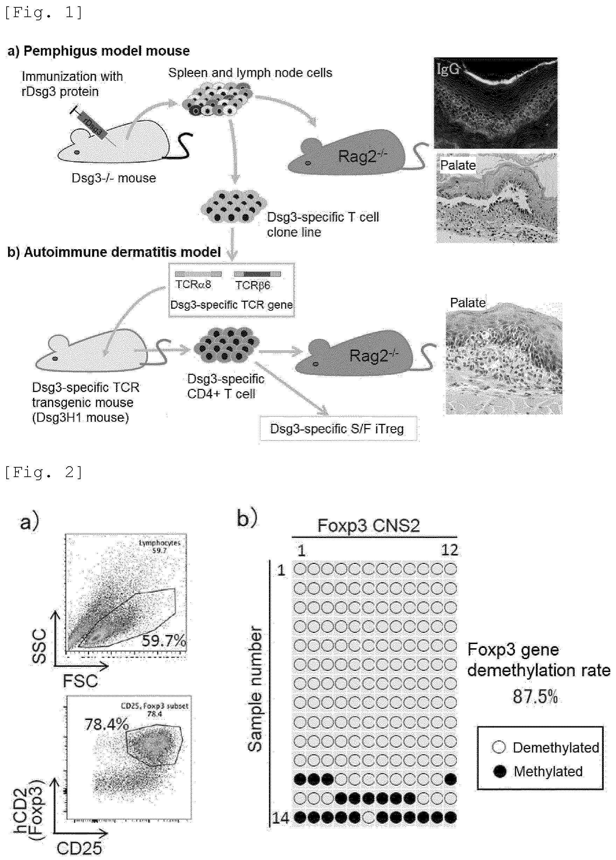

For the production of the inducible regulatory T cells of the present disclosure, a model mouse as shown in FIG. 1 can be used. That is, Dsg3-deficient mice are immunized with Dsg3 protein to generate Dsg3-reactive lymphocytes, and the Dsg3-reactive lymphocytes are administered to Rag2-deficient mice to create a pemphigus model. A Dsg3-specific TCR is cloned from Dsg3-reactive T cells contained in the Dsg3-reactive lymphocytes, and T cells purified from the TCR transgenic mice (Dsg3H1 mice) are administered to Rag2-deficient mice to create a Dsg3-specific dermatitis model mouse.

### (Example 1: Preparation of Dsg3-specific iTregs)

Naive T cells with the phenotype CD4⁺CD25⁻ CD62L^{hi}CD44^{lo}TCRVb6⁺ were sorted from H1 mice, and S/F iTregs were generated. The method is as follows. The purified T cells were stimulated for 3 days in a basal medium containing an anti-CD3 antibody (the antibody at a concentration of 10 ug/ml was added in an amount of 100 µL per well and allowed to stand at room temperature for 60 minutes to immobilize the antibody on the container), hIL-2 (50 U/ml), hTGFβ1 (2.5 ng/ml), retinoic acid (1 µM), and AS2863619 (1 µM). Thereafter, a resting culture was performed for 2 days in a basal medium containing hIL-2 (100 U/ml). After 2 days, the medium was similarly replaced, and a resting culture was further performed for another 2 days. After 2 days, the cells were again stimulated for 2 days in a basal medium containing an anti-CD3 antibody (the antibody at a concentration of 10 µg/ml was added in an amount of 100 µL per well and allowed to stand at room temperature for 60 minutes to immobilize the antibody on the container), hIL-2 (100 U/ml), hTGFβ1 (2.5 ng/ml), retinoic acid (1 µM), AS2863619 (1 µM), and ascorbic acid (10 µg/ml). Thereafter, a resting culture was performed for 2 days in a basal medium containing hIL-2 (100 U/ml). After 2 days, the medium was similarly replaced, and a resting culture was further performed for another 2 days. The expression of Foxp3 and CD25 in the regulatory T cells obtained after 2 days was analyzed by flow cytometry (FIG. 2, left), and the demethylation state of the Treg-specific demethylated region was analyzed by the bisulfite method (FIG. 2, right).

Foxp3 gene demethylation was 87.5%, indicating that functionally stable Dsg3-specific iTregs were successfully prepared.

### (Example 2: Dsg3-specific immunosuppression in vitro)

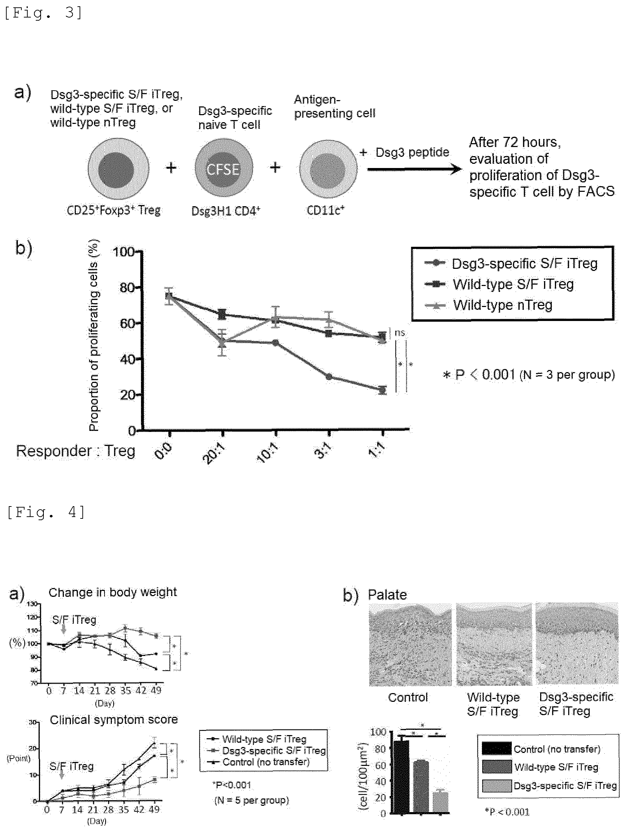

CD4+ naive T cells were labeled with carboxyfluorescein diacetate succinimidyl ester (CFSE, Thermo Scientific) according to the following protocol. Cells suspended in PBS at 1 × 10⁶ cells/mL were incubated with the reagent at 5 µM at room temperature for 5 minutes. The labeling reaction was quenched by adding 500 µl of RPMI medium at 4°C and incubated on ice for 20 minutes. After washing the cells once, 4 × 10⁴ labeled cells were co-cultured in the presence of 2 µM Dsg3H1 peptide with 4 × 10³ CD11c⁺ antigen-presenting cells and with 2 × 10³, 4 × 10³, 1.3 × 10⁴, or 4 × 10⁴ WTS/F iTregs, H1 S/F iTregs, or WT nTregs. For cell proliferation, CFSE was confirmed after 72 hours by flow cytometry. As shown in FIG. 3, H1 S/F iTregs (Dsg3-specific S/F iTregs) exhibited a higher suppressive effect on H1 T cells under Dsg3 peptide stimulation compared to WT (wild-type) nTregs and WT (wild-type) S/F iTregs.

### (Example 3: Dsg3-specific immunosuppression in vivo: dermatitis model)

2 × 10⁵ H1 naive T cells were suspended in 200 µl of PBS and intravenously injected via the tail vein of Rag2^{-/-} mice to create a Dsg3-specific dermatitis model. After one week, 2 × 10⁴ cells of either WT (wild-type) S/F iTregs or H1 S/F iTregs were suspended in 200 µl of PBS and intravenously injected via the tail vein of Rag2^{-/-} mice to observe the therapeutic effect. No iTregs were administered to the control mice. The therapeutic effect was evaluated once a week using the dermatitis score (see Table 1).

### Dermatitis score

**[Table 1]**

| Score | Ears | Tail | Ventral neck and trunk | Back | Face | Limbs | Hair coat |
|---|---|---|---|---|---|---|---|
| 0 | Normal | Normal | Normal | Normal | Normal | Normal | Normal |
| 1 | Erythema | Scale | Erythema (moderate) | Erythema (moderate) | Erythema (moderate) | Erythema (moderate) | |
| 2 | Scale | Crust/erosion (proximal) | Erythema (severe) | Erythema (severe) | Scale | Erythema (severe) | |
| 3 | Crust/erosion | Crust/erosion (whole) | Scale | Scale | Crust/erosion | Scale | Matted |
| 4 | Rim defect | Hair loss | Crust/erosion | Crust/erosion | Hair loss **<** 50% | Crust/erosion | |
| 5 | - | - | Hair loss **<** 50% | Hair loss < 50% | Hair loss > 50% | Hair loss < 50% | |
| 6 | - | - | Hair loss > 50% | Hair loss **>** 50% | | Hair loss > 50% | |

After 7 weeks, the palates of the mice were excised, fixed in 10% formaldehyde for 24 hours, and then dehydrated, defatted, and paraffin-embedded using an automatic embedding device. The paraffin embedding was performed with a paraffin block creation device to create paraffin blocks. Sections with a thickness of 4 µm were cut using a microtome. The sectioned specimens placed on glass slides were spread and dried on a water bath set at 40°C. A deparaffinization operation was performed with xylene, and a xylene removal operation was performed with pure alcohol. After washing with running water, the specimens were transferred to purified water. Staining was performed with a hematoxylin solution^{*1} for 3 to 5 minutes. Color development was performed with 0.05% lithium carbonate by dipping 10 times, and the specimens were washed with running water for 3 minutes. 95% ethanol was passed through, and staining was performed with an eosin alcohol solution^{*2}. Dehydration was performed with anhydrous alcohol. The specimens were cleared with anhydrous xylene and sealed with an automatic sealing device.

### *1, Hematoxylin

Hematoxylin (Merck): 2g
Potassium alum (Sigma) *AIK(SO₄)₂·12H₂O: 50g
Sodium iodate (Wako) NaIO₃: 0.4g
Glycerin (Sigma): 200ml
Purified water: 800ml

### *2, hydrochloric acid-treated eosin

Eosin Y (Merck): 10g
Concentrated hydrochloric acid (Sigma) (HCl): 10ml
Purified water: 1000ml

As shown in FIG. 4, both WT (wild-type) S/F iTregs and H1 S/F iTregs suppressed the clinical symptoms of dermatitis. H1 S/F iTregs showed a greater suppressive effect on dermatitis than WT (wild-type) S/F iTregs. A similar result was observed when comparing the number of lymphocytes infiltrating the skin in pathological tissue, and the control mice had the highest number of infiltrating T cells, while the mice administered with H1S/F iTregs (Dsg3-specific S/F iTregs) had the lowest number of infiltrating T cells.

### (Example 4: Dose dependency: dermatitis model)

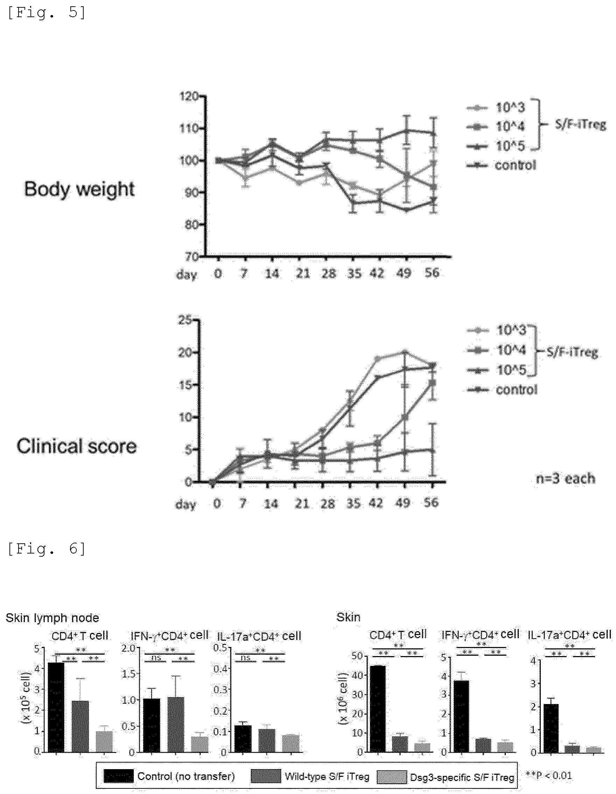

2 × 10⁵ H1 naive T cells were suspended in 200 µl of PBS and intravenously injected via the tail vein of Rag2^{-/-} mice to create a Dsg3-specific dermatitis model. After one week, 1 × 10³, 1 × 10⁴, and 1 × 10⁵ H1S/F iTregs were administered, and the therapeutic effect was observed once a week using the dermatitis score. No iTregs were administered to the control mice. As shown in FIG. 5, the symptoms of dermatitis were lowest in the group administered 1 × 10⁵ cells, followed by the group administered 1 × 10⁴ cells, then the group administered 1 × 10³ cells, and highest in the control. From this result, it was found that the symptoms of dermatitis can be suppressed in a dose-dependent manner by H1S/F iTregs.

### (Example 5: Suppression effect on number of inflammatory cells and production of IFNg and IL-17: dermatitis model)

In the Dsg3-specific dermatitis model, flow cytometric analysis was additionally performed to observe cytokine production. Skin draining lymph nodes were collected, and the skin cell suspension was prepared from the dorsal skin of the mice. The hair was shaved with electric clippers, and the subcutaneous tissue was mechanically removed with forceps. The remaining skin was minced with scissors in 5 ml of RPMI containing 0.25 mg/ml of Liberase TL Research grade (Roche) and 1 g/ml of DNase I (Sigma) in a 6-well plate and incubated at 37°C for 2 hours. After dissociating and collecting the cells, the cells were washed and filtered with a 40 m cell strainer (Falcon).

100 µl of a 200-fold dilution of anti-CD4 antibody and anti-TCRVβ6 antibody in PBS containing 2% FBS was added, and staining was performed on ice for 20 minutes. Using BD Pharmingen Transcription Factor Buffer Set (BD), 100 µl of a cell fixation solution was added, and a reaction was performed on ice for 30 minutes to fix the cells. Thereafter, 100 µl of a 200-fold dilution of anti-IFN-g antibody and anti-IL-17a antibody in wash buffer was added, and staining was performed for 30 minutes. Flow cytometry analysis was performed.

As shown in FIG. 6, in both cases, the T cell count was lowest in H1 S/F iTregs (Dsg3-specific S/F iTregs). In addition, the production of inflammatory cytokines such as IFNγ and IL-17a was also most suppressed in H1 S/F iTregs.

### (Example 6: Long-term survival in dermatitis model mice: after 2 months)

In the Dsg3-specific dermatitis model mice, 2 × 10⁵ H1 S/F iTregs were suspended in 200 ml of PBS and intravenously injected via the tail vein, and after 56 days, the skin draining lymph nodes were collected, 100 µl of a 200-fold dilution of anti-CD4 antibody and anti-hCD2 antibody in PBS containing 2% FBS was added, and staining was performed on ice for 20 minutes. Flow cytometry analysis was performed.

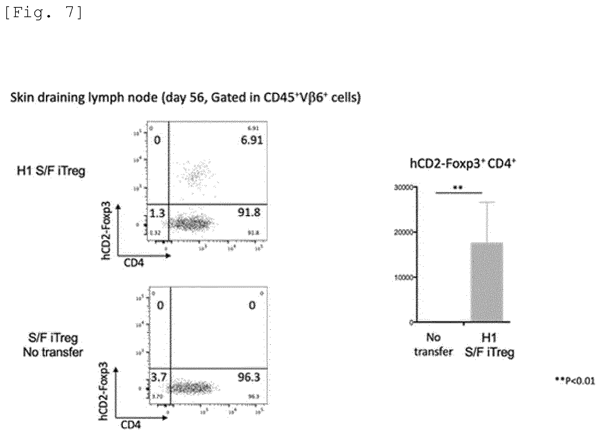

As shown in FIG. 7, CD4⁺Foxp3⁺ cells were observed in the mice to which H1 S/F iTregs were administered, and the survival of H1 S/F iTregs in vivo for 56 days was confirmed.

### (Example 7: Suppressive effect in pemphigus model)

The recombinant extracellular domain of mouse Dsg3 (mDsg3) was diluted in PBS-Ca (0.5 mM CaCl₂ in PBS) to a concentration of 100 µg/ml. On day 0, an emulsion of mDsg3 in the same amount as Complete Freund's Adjuvant (SIGMA, F5881) was prepared using a syringe and 200 µl was administered subcutaneously, on days 7 and 14, an emulsion of mDsg3 in the same amount as Incomplete Freund's Adjuvant (SIGMA, F5506) was prepared using a syringe and 200 µl was administered subcutaneously, and on days 21 and 28, mDsg3 was diluted in PBS-Ca to 50 µg/ml, and 200 µl was administered intraperitoneally to immunize Dsg3^{-/-} mice.

The spleen and skin draining lymph nodes of Dsg3^{-/-} mice immunized with Dsg3 were collected, the spleen was reacted with ACK for 1 minute to cause hemolysis, and the cell suspension was collected. On day 31, 1.5 × 10⁷ cells from the spleen and skin draining lymph nodes were suspended in 200 µl of PBS and administered via tail vein injection to Rag2-/- mice to prepare pemphigus model mice. At the same time, 2 × 10⁵ H1 S/F iTregs were administered via the tail vein, and the suppressive effect on symptoms in the pemphigus model was observed. No iTregs were administered to the control. Blood samples were collected once a week from the bulbar conjunctiva. The titer of anti-Dsg3 IgG in the serum was measured by ELISA using a plate coated with 5 µg/ml of rDsg3 protein. Each serum sample was diluted 1,000-fold with an antibody diluent (prepared with 1% BSA + 0.05% tween in PBS). The serum obtained from PV model mice was used as a positive control for anti-Dsg3 IgG. 50 µl of serum was added to the plate and allowed to stand at room temperature for 1 hour. Thereafter, washing was performed, 50 µl of a solution obtained by diluting an anti-mouse IgG antibody 5,000-fold with an antibody diluent was added, washing was performed at room temperature for 1 hour, 100 µl of an enzyme substrate solution was added and allowed to stand at room temperature for 10 minutes, 100 µl of a reaction stop solution was added and allowed to stand at room temperature for 10 minutes, and absorbance at 450 nm was measured. The ELISA index was calculated using positive and negative controls ((OD450 of sample - OD450 of negative control)/(OD450 of positive control - OD450 of negative control)). The skin symptoms of pemphigus were evaluated once a week using a dermatitis score.

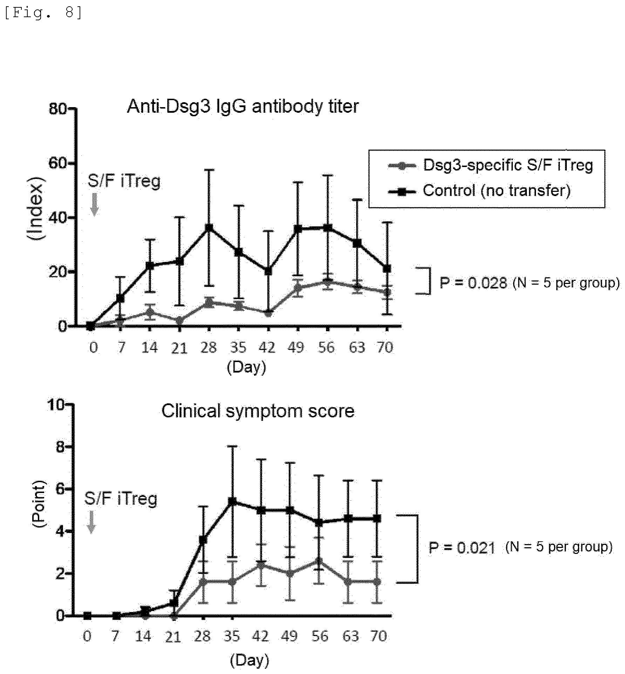

The results are shown in FIG. 8. As shown in FIG. 8, in the H1 S/F iTreg-administered group, the production of Dsg3 antibodies was suppressed compared to the control, and the clinical symptoms of pemphigus were also suppressed.

### (Example 8: Preparation of highly functional and stable human iTregs from T cells derived from pemphigus patients)

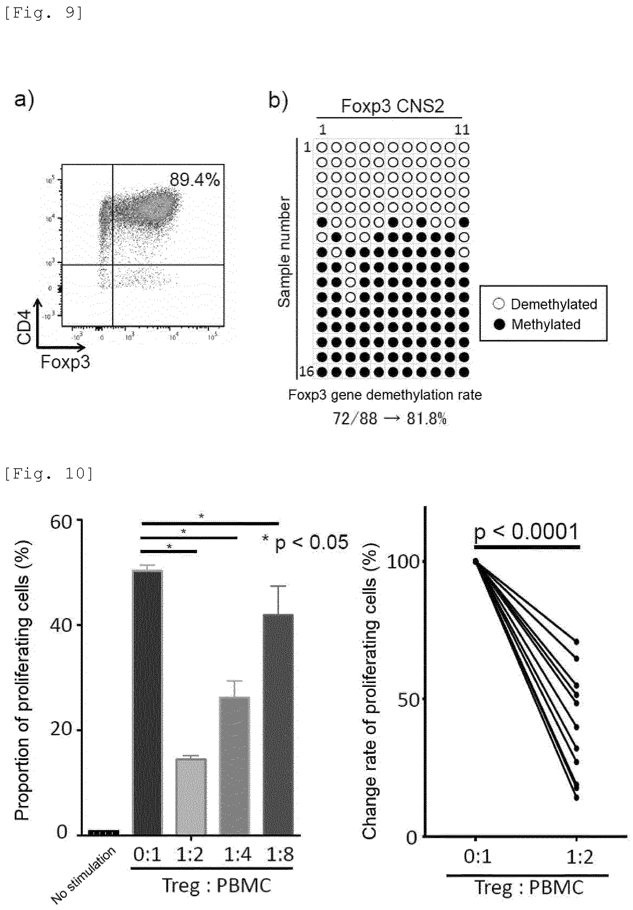

S/F iTregs were prepared from the peripheral blood of pemphigus patients in the same manner as in Example 1. PBMCs were collected from human blood by density gradient centrifugation, the PBMCs were fractionated into CD14-positive cells and CD14-negative cells using beads conjugated with an anti-CD14 antibody, and then, using beads conjugated with an anti-CD4 antibody, the CD14-negative cell fraction was further fractionated into CD4-positive cells and CD4-negative cells. The obtained CD4-positive cells were stimulated for 3 days in a basal medium containing an anti-hCD3 antibody (the antibody at a concentration of 10 ug/ml was added in an amount of 100 µL per well to a 96-well flat-bottom plate and added in an amount of 600 µL per well to a 24-well flat-bottom plate, and then allowed to stand at room temperature for 60 minutes to immobilize the antibody on the container), hIL-2 (50 U/ml), hTGFβ3 (10 ng/ml), AS2863619 (1 µM), and dupilumab (5 µg/ml). Thereafter, a resting culture was performed for 2 days in a basal medium containing hIL-2 (100 U/ml) and AS2863619 (1 µM). After 2 days, the medium was similarly replaced, and a resting culture was further performed for another 2 days. After 2 days, the cells were again stimulated for 2 days in a basal medium containing an anti-hCD3 antibody (the antibody at a concentration of 10 µg/ml was added in an amount of 100 µL per well to a 96-well plate and added in an amount of 600 µL per well to a 24-well flat-bottom plate, and then allowed to stand at room temperature for 60 minutes to immobilize the antibody on the container), hIL-2 (50 U/ml), hTGFβ3 (10 ng/ml), AS2863619 (1 µM), dexamethasone (10 nM), and actosin (20 nM). Thereafter, a resting culture was performed for 2 days in a basal medium containing hIL-2 (100 U/ml) and AS2863619 (1 µM). After 2 days, the medium was similarly replaced, and a resting culture was further performed for another 2 days. The expression of FoxP3 and CD4 in the regulatory T cells obtained after 2 days was analyzed by flow cytometry (FIG. 9, left), and the demethylation state of the Treg-specific demethylated region was analyzed by the bisulfite method (FIG. 9, right).

### (Example 9: Suppressive effect of highly functional and stable iTregs derived from pemphigus patients)

Dsg3-specific immunosuppression by highly functional and stable iTregs derived from pemphigus patients was confirmed in the same manner as in Example 2. Human PBMCs were suspended in a CFSE solution adjusted to 5 µM with MACS buffer, and iTregs were suspended in a Far Red solution adjusted to 1 µM with MACS buffer, and each was stained by leaving it in the dark for 20 minutes. Thereafter, the cells were centrifuged at 500 × g for 5 minutes, and the precipitate was suspended in a 5% human albumin solution and allowed to stand on ice for 5 minutes. Thereafter, the cells were centrifuged at 500 × g for 5 minutes, the supernatant was discarded, PBMCs or iTregs were each suspended at 10⁶ cells/mL in a medium in which human albumin was added to a PRIME-XVT Cell Expansion XSFM serum-free medium so as to have a final concentration of 0.5%. PBMCs were seeded into each well of a 96-well round-bottom plate at 100 µL (10⁵ cells) per well. S/F iTregs were mixed with PBMCs at a ratio ranging from 0 : 1 to 1 : 8, a medium was added to wells where the volume was insufficient to equalize the volume of the medium in each well, and hIL-2 was added so that a final concentration was 10 U/ml. Furthermore, beads conjugated with anti-CD2, CD3, and CD28 antibodies were added at 5 µL per 200 µL of the medium to stimulate the cells, and the cells were cultured at 37°C for 4 days. Thereafter, intracellular CD4 was stained with PE-Cy7 and analyzed by FACS. By gating on Far Red-negative cells to remove Ultra iTregs, the proportion of divided CD4-positive cells was analyzed from the cell population that was both PE-Cy7-positive and CFSE-positive.

The results are shown in FIG. 10. By adding beads conjugated with anti-CD2, CD3, and CD28 antibodies, the proportion of divided CD4-positive cells increased remarkably; however, when iTregs were added, the proportion of divided CD4-positive cells decreased in a cell count-dependent manner, showing a high cell division suppressive effect of the iTregs.

### (Example 10: Clinical trial plan for human pemphigus)

A single-center, open-label, dose-escalation, single-administration phase I clinical trial is planned to evaluate the safety and efficacy of a single intravenous administration of 3 × 10⁷ cells/patient (body weight exceeding 50 kg) or 1 × 10⁸ cells/patient (body weight exceeding 50 kg) of highly functional and stable iTregs derived from pemphigus patients, in patients with steroid treatment-resistant pemphigus vulgaris and pemphigus foliaceus.

The constituent cells of the present product are obtained by converting activated T cells contained in human PBMCs derived from the patient by apheresis into iTregs, which are re-administered to the patient.

The primary endpoint in this trial is to confirm safety (type, frequency, and severity of adverse events) characteristics up to 24 weeks after iTreg administration. The secondary endpoints are the changes from the baseline in the clinical symptom score Pemphigus Disease Area Index (PDAI, an international standard for pemphigus severity), at 24 weeks after iTreg administration, and the changes from the baseline in desmoglein 3 and desmoglein 1 antibody titers up to 24 weeks after iTreg administration.

### (Example 11: Treatment of human pemphigus)

In patients undergoing treatment for pemphigus, symptoms often relapse during steroid tapering. At that time, it is possible to administer highly functional and stable patient-derived iTregs intravenously, either while temporarily increasing the steroid dose or without increasing it. When symptoms worsen, the clinical symptom score Pemphigus Disease Area Index (PDAI) increases. By administering the highly functional and stable iTregs, it is possible to reduce the steroid dose to below the dose used at the time of relapse without an increase in the PDAI. In addition, a decrease in anti-desmoglein 3 or 1 antibody titers is expected by administration of highly functional and stable iTregs. In cases where antibody titers decreased in association with the administration, antibody titers can also be monitored on a monthly basis, and the sustained efficacy of the administered highly functional and stable iTregs can be evaluated. In such cases, when a future re-increase in the antibody titer and subsequently a re-increase in the PDAI are confirmed, the highly functional and stable iTregs can be re-administered to reduce the patient's symptoms again.

### (Example 12: Treatment with Dsg3-specific S/F iTregs)

In the present example, treatment with Dsg3-specific S/F iTregs was examined.

### (Biochemical test)

### 1) Method

Lymphocytes were prepared from the spleen and lymph nodes of Dsg3KO mice immunized with recombinant Dsg3 protein, and a pemphigus model was created by administering 1.5 × 10^7/body via the tail vein to Rag2KO mice. After one week, 1.0 × 10^7 Dsg3H1-specific S/F iTregs were administered. The control had no cell administration. The treatment group and the control group each had N = 5.

The total B cell count was analyzed by staining with B220 and performing FACS after counting the number of spleen and lymph node cells 6 weeks after creation of the pemphigus model mice.

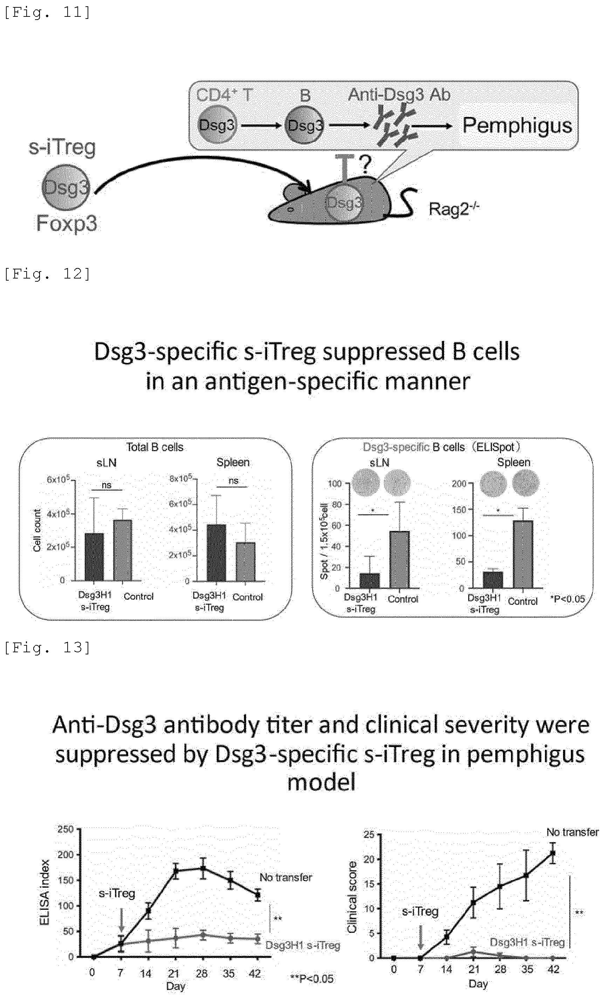

The Dsg3-specific B cell count was measured in the spleen and lymph nodes, as described above, by an ELISpot method (FIG. 11).

### 2) Results

Treatment with Dsg3-specific S/F iTregs did not affect the total B cell count compared to the control group, but significantly reduced Dsg3-specific B cell count (FIG. 12).

### 3) Discussion

The Dsg3-specific S/F iTregs suppressed B cells in a Dsg3-specific manner.

### (Clinical score)

### 1) Method

The peripheral blood of the mice treated by the above method was collected weekly from the eye, and the anti-Dsg3 antibody titer was measured by the ELISA method. Regarding the clinical score, the extent of skin symptoms and hair loss throughout the body was evaluated once a week using a dermatitis score.

### 2) Results

The Dsg3-specific S/F iTregs suppressed both anti-Dsg3 antibodies and clinical symptoms.

### 3) Discussion

The Dsg3-specific S/F iTregs suppressed B cells in a Dsg3-specific manner, further suppressed Dsg3 antibody production and clinical symptoms of pemphigus, and therefore, were considered to be useful as an antigen-specific therapy (FIG. 13).

### (Note)

Although the present disclosure has been illustrated using preferred embodiments of the present disclosure as described above, it is understood that the scope of the present disclosure should be interpreted only by the claims. It is understood that the patents, patent applications, and other documents cited in the present specification are to be incorporated by reference in the present specification in the same manner as the contents are specifically described in the present specification. The present application claims priority to Japanese Patent Application No. 2023-17733, filed on February 8, 2023 to the Japan Patent Office, the entire contents of which are incorporated herein by reference as necessary.

### Industrial Applicability

The cells or cell population of the present disclosure can be used for treating or preventing pemphigus. In addition, it is possible to stably induce regulatory T cells having high functionality from peripheral T cells by the method for producing a pharmaceutical composition of the present disclosure, and application to the medical field can be expected.

## Claims

1. A pharmaceutical composition for treating or preventing pemphigus, the pharmaceutical composition comprising, as an active ingredient, an inducible regulatory T cell having at least one characteristic selected from the group consisting of CTLA4-positive, NT5E-positive, ITGAE (CD103)-positive, AREG-positive, CD172g-positive, and CD26-positive.

2. A pharmaceutical composition for treating or preventing pemphigus, the pharmaceutical composition comprising, as an active ingredient, an inducible regulatory T cell that is NT5E-positive.

3. A pharmaceutical composition for treating or preventing pemphigus, the pharmaceutical composition comprising, as an active ingredient, an inducible regulatory T cell that is ITGAE (CD103)-positive.

4. A pharmaceutical composition for treating or preventing pemphigus, the pharmaceutical composition comprising, as an active ingredient, an inducible regulatory T cell that is AREG-positive.

5. A pharmaceutical composition for treating or preventing pemphigus, the pharmaceutical composition comprising, as an active ingredient, an inducible regulatory T cell that is CD172g-positive.

6. A pharmaceutical composition for treating or preventing pemphigus, the pharmaceutical composition comprising, as an active ingredient, an inducible regulatory T cell that is CD26-positive.

7. A pharmaceutical composition for treating or preventing pemphigus, the pharmaceutical composition comprising, as an active ingredient, an inducible regulatory T cell that is CTLA4-positive.

8. The pharmaceutical composition according to any one of claims 1 to 7, wherein the inducible regulatory T cell is at least CTLA4-positive and FoxP3-positive.

9. The pharmaceutical composition according to any one of claims 1 to 8, wherein the inducible regulatory T cell is at least CD172g-positive and/or CD26-positive.

10. The pharmaceutical composition according to any one of claims 1 to 9, wherein the CNS2 region of the FOXP3 gene of the inducible regulatory T cell is demethylated.

11. The pharmaceutical composition according to any one of claims 1 to 10, wherein the inducible regulatory T cell is CD4-positive or CD8-positive.

12. The pharmaceutical composition according to any one of claims 1 to 11, wherein the inducible regulatory T cell is obtained from or derived from a human peripheral blood T cell or a human tissue-derived T cell.

13. The pharmaceutical composition according to any one of claims 1 to 12, wherein the inducible regulatory T cell is obtained by a method including:
(a) stimulating CD4-positive T cells or CD8-positive T cells in human peripheral blood with a first basal medium for about 1 to about 5 days;
(b) subjecting the cells obtained in the step (a) to a resting culture in a medium containing IL-2 for at least about 1 to about 3 days;
(c) stimulating the cells obtained in the step (b) with a second basal medium for about 1 to about 5 days; and
(d) subjecting the cells obtained in the step (c) to a resting culture in a medium containing IL-2 for at least about 1 to about 3 days.

14. The pharmaceutical composition according to any one of claims 1 to 13, wherein the pharmaceutical composition contains, as an active ingredient, a cell population containing the inducible regulatory T cells, and the cell population has a proportion of about 50% or more of cells each having at least one characteristic selected from the group consisting of FoxP3-positive, CTLA4-positive, NT5E-positive, ITGAE (CD103)-positive, AREG-positive, CD172g-positive, and CD26-positive.

15. A pharmaceutical composition for treating or preventing pemphigus, the pharmaceutical composition comprising, as an active ingredient, a cell population containing inducible regulatory T cells,
wherein a proportion of NT5E-positive cells in the cell population is about 50% or more.

16. A pharmaceutical composition for treating or preventing pemphigus, the pharmaceutical composition comprising, as an active ingredient, a cell population containing inducible regulatory T cells,
wherein a proportion of ITGAE (CD103)-positive cells in the cell population is about 50% or more.

17. A pharmaceutical composition for treating or preventing pemphigus, the pharmaceutical composition comprising, as an active ingredient, a cell population containing inducible regulatory T cells,
wherein a proportion of AREG-positive cells in the cell population is about 50% or more.

18. A pharmaceutical composition for treating or preventing pemphigus, the pharmaceutical composition comprising, as an active ingredient, a cell population containing inducible regulatory T cells,
wherein a proportion of CD172g-positive cells in the cell population is about 50% or more.

19. A pharmaceutical composition for treating or preventing pemphigus, the pharmaceutical composition comprising, as an active ingredient, a cell population containing inducible regulatory T cells,
wherein a proportion of CD26-positive cells in the cell population is about 50% or more.

20. A pharmaceutical composition for treating or preventing pemphigus, the pharmaceutical composition comprising, as an active ingredient, a cell population containing inducible regulatory T cells,
wherein a proportion of CTLA4-positive cells in the cell population is about 50% or more.

21. The pharmaceutical composition according to any one of claims 14 to 20, wherein proportions of at least the CTLA4-positive cells and FoxP3-positive cells in the cell population are each about 50% or more.

22. The pharmaceutical composition according to any one of claims 14 to 21, wherein proportions of at least the CD172g-positive cells and/or CD26-positive cells in the cell population are each about 50% or more.

23. The pharmaceutical composition according to any one of claims 14 to 22, wherein a proportion of cells having the at least one characteristic in the cell population is about 60% or more.

24. The pharmaceutical composition according to any one of claims 14 to 23, wherein a proportion of cells having the at least one characteristic in the cell population is about 80% or more.

25. The pharmaceutical composition according to any one of claims 14 to 24, wherein a proportion of FoxP3 strong-positive cells in the cell population is about 50% or more.

26. The pharmaceutical composition according to any one of claims 1 to 25, wherein the cell population contains about 90% or more T cells.

27. The pharmaceutical composition according to any one of claims 1 to 26, wherein the inducible regulatory T cells are contained in an amount of at least about 1 × 10³ cells per administration.

28. The pharmaceutical composition according to any one of claims 1 to 27, wherein the pharmaceutical composition is administered by injection.

29. The pharmaceutical composition according to any one of claims 1 to 28, wherein when the pharmaceutical composition is administered to a patient and is ineffective or insufficient, the pharmaceutical composition is additionally administered to the patient.

30. The pharmaceutical composition according to any one of claims 1 to 29, wherein the inducible regulatory T cell specifically responds to desmoglein 3 (Dsg3).

31. A pharmaceutical composition comprising, as an active ingredient, an inducible regulatory T cell that has at least one characteristic selected from the group consisting of CTLA4-positive, NT5E-positive, ITGAE (CD103)-positive, AREG-positive, CD172g-positive, and CD26-positive, and specifically responds to desmoglein 3 (Dsg3).
